# EUROPÄISCHE PATENTSCHRIFT

(11) **EP 1 299 342 B1**
(45) Veröffentlichungstag und Bekanntmachung des Hinweises auf die Patenterteilung: **02.05.2007**
(21) Anmeldenummer: 01962840.3
(22) Anmeldetag: 06.07.2001
(51) Int. Cl.: C07C 209/26, C07C 213/02, C07D 311/20, C07D 311/58

(54) **VERFAHREN ZUR HERSTELLUNG VON 3,3-DIARYLPROPYLAMINEN**
METHOD FOR PRODUCING 3,3-DIARYLPROPYLAMINES
PROCEDE DE PRODUCTION DE 3,3-DIARYLPROPYLAMINES

(30) Priorität: 07.07.2000 DE 10033016
(43) Veröffentlichungstag der Anmeldung: 09.04.2003
(73) Patentinhaber: SCHWARZ PHARMA AG, 40789 Monheim/Rhld. (DE)
(72) Erfinder: DONSBACH, Martin, 50321 Brühl (DE); EILBRACHT, Peter, 44229 Dortmund (DE); BUSS, Christian, 01129 Dresden (DE); SCHMIDT, Andreas, 44309 Dortmund (DE)
(74) Vertreter: Bausch, Thorsten
(86) Internationale Anmeldenummer: PCT/EP2001/007803
(87) Internationale Veröffentlichungsnummer: WO 2002/004399

(56) Entgegenhaltungen:
- WO-A-94/11337
- WO-A-97/44329
- WO-A-98/29402
- WO-A-99/58478

## Beschreibung

Die Erfindung betrifft ein neues Verfahren zur Herstellung von substituierten 3,3-Diarylpropylaminderivaten. Insbesondere betrifft sie die Herstellung solcher Verbindungen durch Hydrocarbonylierung/Hydroformylierung mit anschließender reduktiver Aminierung.

Die vorgenannten 3,3-Diarylpropylaminderivate werden zur Behandlung von Harndrang-Inkontinenz und anderen spasmogenen Leiden eingesetzt (vgl. WO 99/58478). Die dort beschriebenen Verfahren zur Herstellung der 3,3-Diphenylpropylaminderivate sind zumeist mehrstufig und erfordern zur Herstellung optisch aktiver Verbindungen zumeist eine Enantiomerentrennung.

Der Erfindung liegt daher die Aufgabe zugrunde, neue Verfahren zur Herstellung substituierter 3,3-Diarylpropylaminderivate bereitzustellen, die einfacher sind als die im Stand der Technik beschriebenen, d.h. weniger Stufen umfassen, und die überdies eine stereoselektive Synthese der Zielverbindungen ermöglichen.

Gegenstand der Erfindung ist ein Verfahren zur Herstellung von 3,3-Diarylpropylaminen der allgemeinen Formel I 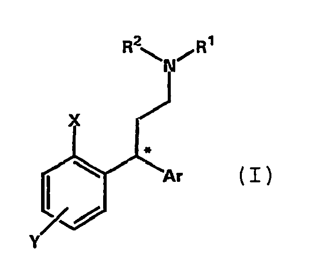 worin
Ar für einen substituierten oder unsubstituierten Arylrest steht,
X für OH oder OR³ steht,
Y für Cl, Br, I, CN, CHO, CH₂OR, COOH, COOR, worin R für C₁-C₁₀-Alkyl oder einen substituierten oder unsubstituierten Arylrest steht, oder C₁-C₁₀-Alkyl steht,
R¹, R² für C₁-C₁₀-Alkyl oder C₃-C₈-Cycloalkyl steht, wobei R¹ und R² unter Bildung einer cyclischen Struktur verknüpft sein können,
und wobei R³ für einen Rest steht, der von einer der folgenden Verbindungen abgeleitet ist:
(i) den Aminosäuren D-Prolin, L-Prolin, D-Alanin, L-Alanin, D-Asparagin, L-Asparagin, D-Asparaginsäure, L-Asparaginsäure, D-Glutamin, L-Glutamin, D-Glutaminsäure, L-Glutaminsäure, D-Phenylalanin, L-Phenylalanin, D-Histidin, L-Histidin, D-Leucin, L-Leucin, D-Serin, L-Serin, L-Threonin, D-Threonin, D-Tryptophan, L-Tryptophan, D-Tyrosin, L-Tyrosin, D-Valin, L-Valin, D-Cystein, L-Cystein, D-Methionin, L-Methionin, D-Isoleucin, L-Isoleucin, oder den Alkoholen, die sich aus diesen Aminosäuren durch Reduktion der Carbonsäurefunktion zur Hydroxymethyleneinheit ergeben,
(ii) den Aminosäuren N-Diphenylphosphanyl-D-alanin, N-Diphenylphosphanyl-L-alanin, N-Diphenylphosphanyl-D-prolin, N-Diphenylphosphanyl-L-prolin, N-Diphenylphosphanyl-D-phenylalanin, N-Diphenylphosphanyl-L-phenylalanin, sowie den Alkoholen, die sich aus diesen Aminosäuren durch Reduktion der Carbonsäurefunktion zur Hydroxymethyleneinheit ergeben,
(iii) α-Hydroxycarbonsäurederivaten der allgemeinen Formel 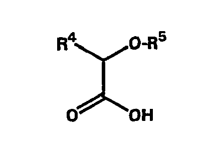 jeweils in Form beider optischen Antipoden, worin R⁴ für eine lineare oder verzweigte C₁-C₁₀-Alkylgruppe oder Cycloalkylgruppe oder einen substituierten oder unsubstituierten Arylrest und R⁵ für C₁-C₁₀-Alkyl, Cycloalkyl, Acyl, Alkoxycarbonyl, Benzoyl, Diphenylphosphanyl, Dicyclohexylphosphanyl oder Diarylphosphanyl steht,
(iv) den Verbindungen der allgemeinen Formeln 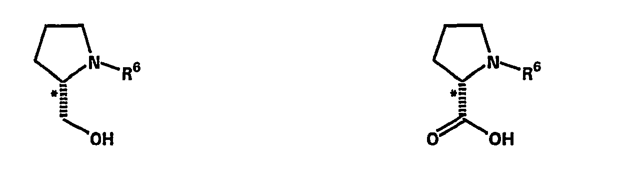 worin R⁶ für einen Substituenten ausgewählt aus der Gruppe bestehend aus PPh₂, P(C₆H₁₁)₂, P(Aryl)₂, Alkyl, Acyl, Alkoxycarbonyl, Benzoyl, Arylcarbonyl, Diarylphosphanyl und Dicyclohexylphosphanyl steht, und deren Stereoisomere,
(v) den Verbindungen der allgemeinen Formeln 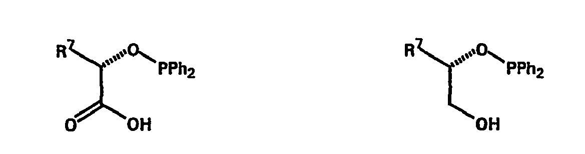 worin R⁷ für eine lineare oder verzweigte C₁-C₁₀-Alkylgruppe oder einen substituierten oder unsubstituierten Arylrest steht, und Stereoisomere davon,
(vi) den Säuren
   (R)-Acetoxyphenylessigsäure,
   (R)- und (S)-1,4-Benzodioxan-2-carbonsäure,
   (R)-(-)- und (S)-(+)-Hexahydro-acetoxymandelsäure,
   (2R,3S)-2,3-O-Isopropyliden-2,3-dihydroxybuttersäure und deren Stereoisomere,
   (+)- und (-)-Menthyloxyessigsäure,
   (R)- und (S)-3-Phenyl-2-acyloxypropionsäure,
   (R)- und (S)-Acetoxymandelsäure,
   (R)- und (S)-α-Methoxy-α-trifluormethylphenylessigsäure,
   (S)-(+)-alpha-Methoxyphenylessigsäure,
   (R)- und (S)-5-Oxo-tetrahydrofuran-2-carbonsäure, sowie den Alkoholen, die sich aus diesen Säuren durch
   Reduktion der Carbonsäurefunktion zur Hydroxymethyleneinheit ergeben,
(vii) Verbindungen der allgemeinen Formel 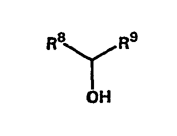 worin R⁸ für einen substituierten oder unsubstituierten Arylrest und R⁹ für Wasserstoff oder für einen linearen oder verzweigten C₁-C₁₀-Alkylrest steht,
(viii) α-Naphthol, β-Naphthol oder (R)- oder (S)-1-(9-Anthryl)-2,2,2-trifluoroethanol,
(ix) 2-Methylamino-1-phenyl-propan-1-ol (Ephedrin) aller stereomerer Formen,
   oder R³ für einen der folgenden Reste steht:
(x) Phosphitreste der allgemeinen Formel -P(OR¹⁰)(OR¹¹), worin R¹⁰ und R¹¹ gleich oder verschieden sein können und für einen gegebenenfalls polycyclischen oder verbrückten Arylrest stehen,
(xi) C₁-C₁₀-Alkyl, verzweigt oder linear,
(xii) Acyl,
(xiii) Benzyl oder substituierte Benzylreste,
dadurch gekennzeichnet, dass man Verbindungen der allgemeinen Formel (II) 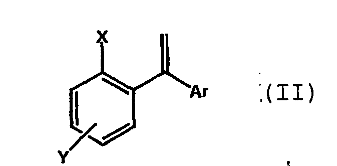 worin X, Y und Ar wie voranstehend definiert sind, mit Kohlenmonoxid (CO) und Wasserstoff (H₂) in Gegenwart eines Übergangsmetallkatalysators, wie im Anspruch 1 definiert, miteinander umsetzt und die resultierenden Oxoverbindungen in Gegenwart eines Übergangsmetallkatalysators in eine Eintopfreaktion direkt mit einem Amin der allgemeinen Formel HNR¹R², worin R¹ und R² wie oben definiert sind, umsetzt.

In der Formel I steht der Substituent X für Wasserstoff (H), Hydroxy (OH) oder die Gruppe OR³.

Der Substituent R³ kann dabei die für die Substituenten R und R' in Patentanspruch 1 der WO 99/58478 angegebene Bedeutung haben.

Vorzugsweise steht R³ für einen Rest, der sich von den folgenden Verbindungen und Alkoholen ableitet:
(i) den Aminosäuren D-Prolin, L-Prolin, D-Alanin, L-Alanin, D-Asparagin, L-Asparagin, D-Asparaginsäure, L-Asparaginsäure, D-Glutamin, L-Glutamin, D-Glutaminsäure, L-Glutaminsäure, D-Phenylalanin, L-Phenylalanin, D-Histidin, L-Histidin, D-Leucin, L-Leucin, D-Serin, L-Serin, L-Threonin, D-Threonin, D-Tryptophan, L-Tryptophan, D-Tyrosin, L-Tyrosin, D-Valin, L-Valin, D-Cystein, L-Cystein, D-Methionin, L-Methionin, D-Isoleucin, L-Isoleucin, oder den Alkoholen, die sich aus diesen Aminosäuren durch Reduktion der Carbonsäurefunktion zur Hydroxymethyleneinheit ergeben.
   Bei diesen Aminosäureresten erfolgt die Anbindung an das Sauerstoffatom gemäß der Formel -O-R³ über die OH-Gruppe der vorgenannten Aminosäuren, wie das in dem nachfolgenden Formelschema dargestellt ist: 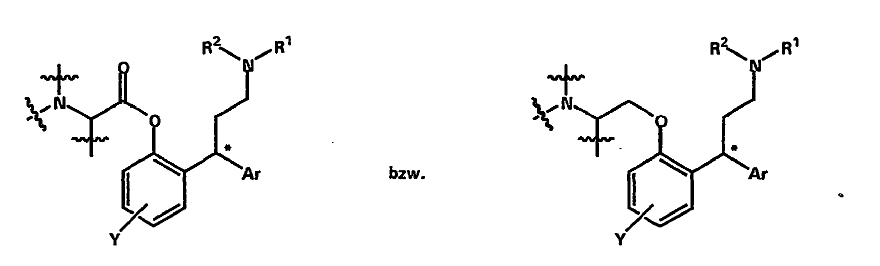
(ii) Weiterhin kann sich der Substituent R³ von den folgenden Aminosäurederivaten ableiten:
   den Aminosäuren N-Diphenylphosphanyl-D-alanin, N-Diphenylphosphanyl-L-alanin, N-Diphenylphosphanyl-D-prolin, N-Diphenylphosphanyl-L-prolin, N-Diphenylphosphanyl-D-phenylalanin, N-Diphenylphosphanyl-L-phenylalanin, sowie den Alkoholen, die sich aus diesen Aminosäuren durch Reduktion der Carbonsäurefunktion zur Hydroxymethyleneinheit ergeben.
(iii) Gemäß einer weiteren Ausführungsform kann sich R³ von den folgenden α-Hydroxycarbonsäuren ableiten:
   α-Hydroxycarbonsäurederivaten der allgemeinen Formel 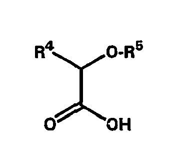 jeweils in Form beider optischen Antipoden, worin R⁴ für eine lineare oder verzweigte C₁-C₁₀-Alkylgruppe oder Cycloalkylgruppe oder einen substituierten oder unsubstituierten Arylrest und R⁵ für C₁-C₁₀-Alkyl, Cycloalkyl, Acyl, Alkoxycarbonyl, Benzoyl, Diphenylphosphanyl, Dicyclohexylphosphanyl oder Diarylphosphanyl steht.
   Bevorzugte Beispiele sind (R)- und (S)-Acetoxyphenylessigsäure, (S)-(+)- und R-(-)-alpha-Methoxyphenylessigsäure, (R)- und (S)-3-Phenyl-2-acyloxypropionsäure.
(iv) R³ kann sich weiterhin von den nachstehend dargestellten Carbonsäuren bzw. Alkoholen ableiten: 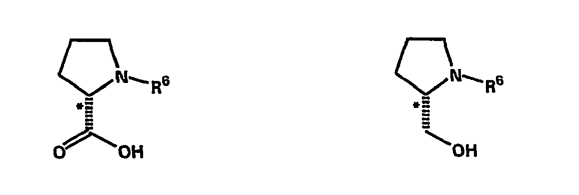 worin R⁶ für einen Substituenten, ausgewählt aus der Gruppe bestehend aus PPh₂, Alkyl, Acyl, Alkoxycarbonyl, Benzoyl, Arylcarbonyl, Diphenylphosphanyl, Diarylphosphanyl, Dicyclohexylphosphanyl steht, und Stereoisomere davon.
(v) Weiterhin kann sich R³ von den nachstehend dargestellten Carbonsäuren bzw. Alkoholen ableiten: 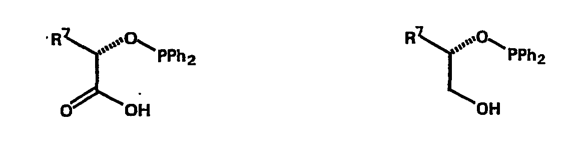 worin R⁷ für eine lineare oder verzweigte C₁-C₁₀-Alkylgruppe oder einen substituierten oder unsubstituierten Arylrest steht, und Stereoisomere davon.
(vi) Weiterhin kann sich der Substituent R³ von einer der nachfolgend dargestellten Carbonsäuren ableiten:
   1. (R)-Acetoxyphenylessigsäure oder das Enantiomer 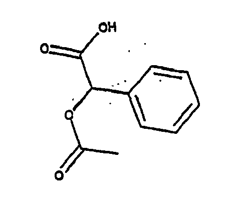
   2. (R)-1,4-Benzodioxan-2-carbonsäure oder das Enantiomer 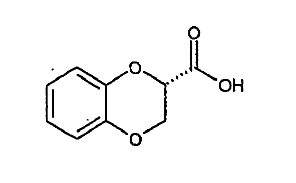
   3. (R)-(-)-Hexahydroacetoxymandelsäure oder das Enantiomer 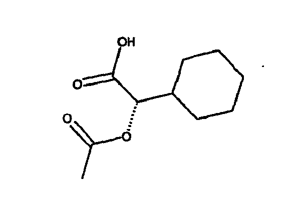
   4. (2R,3S)-2,3-O-Isopropyliden-2,3-dihydroxy-buttersäure oder das Enantiomer 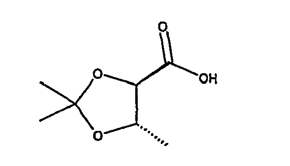
   5. (+)-Menthyloxyessigsäure oder das Enantiomer 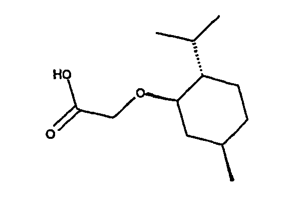
   6. (S)-(+)-alpha-Methoxyphenylessigsäure oder das Enantiomer 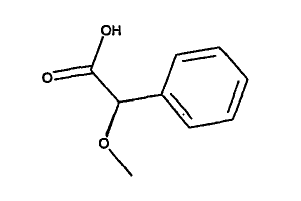
   7. (R)-5-Oxo-tetrahydrofuran-2-carbonsäure oder das Enantiomer 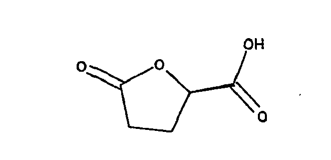
   8. (R)-3-Phenyl-2-acyloxypropionsäure oder das Enantiomer 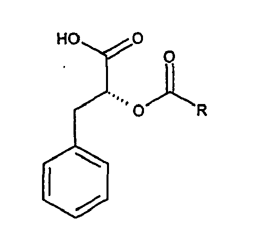 worin R wie voranstehend definiert ist.
   9. (R)-α-Methoxy-α-trifluormethylphenylessigsäure oder das Enantiomer 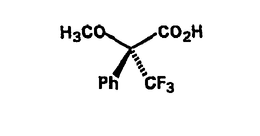 Sämtliche der vorgenannten Säuren besitzen ein oder mehrere Asymmetriezentren und werden optisch aktiv eingesetzt.

   Die Anbindung der Säuren an das Sauerstoffatom gemäß der Formel -O-R³ erfolgt wiederum wie bei den anderen zuvor genannten Carbonsäuren bzw. Alkoholen über die OH-Gruppe, so dass allgemein Ester bzw. Ether mit der folgenden Struktur resultieren: 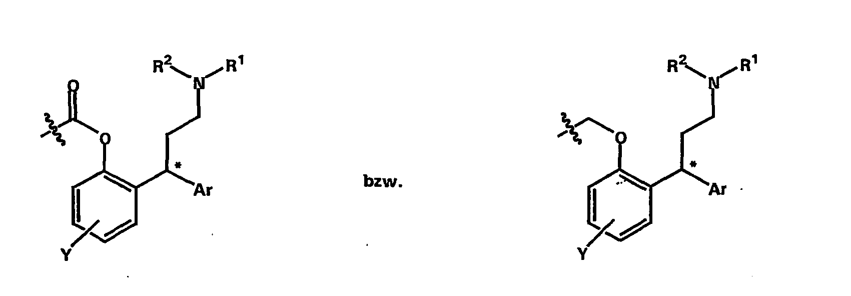
(vii) Der Rest R³ kann sich weiterhin von Verbindungen der allgemeinen Formel 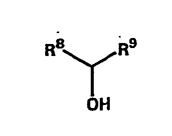 ableiten, worin R⁸ für einen substituierten oder unsubstituierten Arylrest und R⁹ für Wasserstoff oder für einen linearen oder verzweigten C₁-C₁₀-Alkylrest steht.
   Wiederum erfolgt die Anbindung des Substituenten R³ über das Sauerstoffatom, so dass Arylether entstehen.
(viii) Der Substituent R³ kann sich weiterhin von α-Naphthol, β-Naphthol oder (R)- oder (S)-1-(9-Anthryl)-2,2,2-trifluorethanol ableiten.
   Auch hier erfolgt die Anbindung über das Sauerstoffatom.
(ix) Gemäß einer weiteren Ausführungsform kann sich R³ von Ephedrin, d.h. 2-Methylamino-1-phenylpropan-1-ol, ableiten. Diese Verbindung ist chiral und im Rahmen der vorliegenden Erfindung sollen sämtliche stereoisomere Formen mitumfasst werden.
   Die Anbindung von R³ erfolgt wiederum über das Sauerstoffatom, so dass eine Etherstruktur resultiert.
(x) Schließlich kann R³ auch für einen Phosphitrest der allgemeinen Formel -P(OR¹⁰)(OR¹¹) stehen, worin R¹⁰ und R^{11'} gleich oder verschieden sein können und für einen gegebenenfalls polycyclischen oder verbrückten Arylrest stehen.
   Bevorzugte Beispiele sind Phosphitreste der Formeln 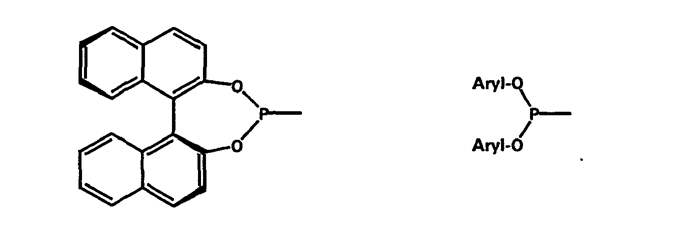 Weiterhin kann R³ für:
(xi) C₁-C₁₀-Alkyl, linear oder verzweigt,
(xii) Acyl,
(xiii) Benzyl oder substituierte Benzylreste stehen.

Der Substituent Y gemäß der allgemeinen Formel I steht für Cl, Br, I, CN, COOH, COOR, CHO, CH₂OR oder C₁-C₁₀-Alkyl.

In der Gruppe COOR bzw. CH₂OR steht der Substituent R für eine lineare oder verzweigte C₁-C₁₀-Alkylgruppe oder einen substituierten oder unsubstituierten Arylrest.

Der Substituent Y ist so gewählt, dass er durch einfache, allgemein bekannte Verfahren in eine Hydroxymethylgruppe umgewandelt werden kann. Beispielsweise wird eine Ester- oder Carbonsäuregruppe direkt reduziert, ein Halogenid kann über eine Grignard-Zwischenstufe in die entsprechende Carbonsäure umgewandelt und anschließend reduziert werden, und ein Nitril kann zunächst beispielsweise zur Carbonsäure hydrolysiert und anschließend zu einer Hydroxymethylgruppe reduziert werden.

Durch diese Umwandlung des Substituenten Y in eine Hydroxymethylgruppe können die in der WO 99/58478 beschriebenen 3,3-Diphenylpropylaminderivate in einfacher Weise hergestellt werden. Dort sind auch entsprechende Verfahren zur Umwandlung des Substituenten Y in eine Hydroxymethylgruppe beschrieben.

Gemäß einer bevorzugten Ausführungsform des erfindungsgemäßen Verfahrens steht Y für eine Estergruppe, die sich in Paraposition zu dem Substituenten X befindet.

Schließlich stehen die Substituenten R¹ und R², die gleich oder verschieden sein können, für eine C₁-C₁₀-Alkylgruppe oder eine C₃-C₈-Cycloalkylgruppe, wobei R¹ und R² unter Bildung einer cyclischen Struktur miteinander verknüpft sein können. Diese cyclische Struktur kann Heteroatome, wie z.B. Stickstoff, Sauerstoff, etc., enthalten, so dass R¹ und R² zusammen mit dem Stickstoffatom, an das sie gebunden sind, beispielsweise einen Morpholin-Rest bilden können.

Gemäß einer bevorzugten Ausführungsform des erfindungsgemäßen Verfahrens stehen R¹ und R² für Isopropyl und Ar für Phenyl.

Im Rahmen der vorliegenden Erfindung bezeichnet der Begriff "Alkyl" eine lineare oder verzweigte Kohlenwasserstoffkette mit vorzugsweise 1 bis 10 C-Atomen. Beispiele für solche Alkylgruppen umfassen Methyl, Ethyl, Propyl, Isopropyl, Butyl, Isobutyl, t-Butyl und entsprechende Isomere von Pentyl und Hexyl, wobei die Isopropylgruppe bevorzugt ist.

Der Begriff "Cycloalkyl" bzw. "Cycloalkylgruppe" beschreibt cyclische Kohlenwasserstoffreste mit vorzugsweise 3 bis 10 Kohlenstoffatomen, die gegebenenfalls substituiert sein können.

Im Rahmen der vorliegenden Erfindung werden unter Substituenten dem Fachmann an sich bekannte Gruppen wie Alkyl, Alkoxy, Halogen (Fluor, Chlor, Brom, Iod), Nitro und dergleichen verstanden.

Der Begriff "substituiertes oder unsubstituiertes Benzyl" beschreibt eine Benzylgruppe, deren Phenylring gegebenenfalls ein- oder mehrfach substituiert ist. Substituierte Benzylgruppen sind vorzugsweise 4-Methylbenzyl, 4-Methoxybenzyl, 4-Nitrobenzyl, 2-Nitrobenzyl, 4-Chlorbenzyl und 2-Chlorbenzyl.

Der Begriff "Alkylcarbonyl" beschreibt Gruppen der Formel R-C(=O), worin R für eine Alkylgruppe steht. Bevorzugte Alkylcarbonylgruppen sind Acetyl, Propionyl, Isobutyryl, Valeroyl und Pivaloyl.

Der Begriff "Cycloalkylcarbonyl" beschreibt eine Gruppe der Formel R-C(=O), worin R für eine Cycloalkylgruppe steht.

Der Begriff "Aryl" bezeichnet einen aromatischen Kohlenwasserstoffrest, wie z.B. Phenyl (C₆H₅-), Naphthyl (C₁₀H₇-) und Anthryl (C₁₄H₉-). Bevorzugt werden Phenyl- und Napthylgruppen, insbesondere Phenylgruppen, wobei diese Gruppen einfach oder mehrfach substituiert sein können. Gegebenenfalls können zwei oder mehrere Arylreste zur Bildung polycyclischer Strukturen miteinander verbrückt bzw. kondensiert sein.

Der Begriff "Benzoyl" beschreibt Acylgruppen der Formel -C(=O)-Ph, wobei der Phenylring wiederum einfach oder mehrfach substituiert sein kann. Beispiele für solche substituierten Acylgruppen umfassen 4-Methoxybenzoyl, 2-Methoxybenzoyl, 4-Chlorbenzoyl, 2-Chlorbenzoyl, 4-Nitrobenzoyl und 2-Nitrobenzoyl.

Der Begriff "Alkoxycarbonyl" steht für R-OC(=O)-Gruppen, worin R für eine Alkylgruppe steht. Bevorzugte Alkoxycarbonylgruppen sind Methoxycarbonyl, Ethoxycarbonyl, Propoxycarbonyl, t-Butyloxycarbonyl und Benzyloxycarbonyl sowie alicyclische Alkoxycarbonylgruppen.

Der Begriff "Aminosäure" bzw. "Aminosäurerest" beschreibt Reste, welche sich von natürlich vorkommenden oder synthetischen Aminosäuren (wobei jeweils alle optischen Antipoden eingeschlossen sind) ableiten. Bevorzugte Aminosäurereste sind Valyl, Leucyl, Isoleucyl, Phenylalanyl, Prolyl, Seryl, Threonyl, Methionyl und Hydroxyprolyl.

Der Aminosäurerest kann durch eine geeignete Gruppe substituiert sein. Solche substituierten Aminosäurereste sind beispielsweise N-Benzoylprolyl, N-tert.-Butoxycarbonylprolyl, N-Alkyl, N-Acyl oder N-Diphenylphosphanylprolyl.

Das erfindungsgemäße Verfahren beruht auf der Hydrocarbonylierung/Hydroformylierung von Verbindungen der allgemeinen Formel 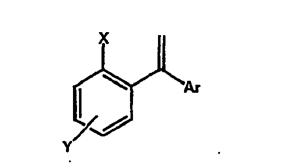 worin X, Y und Ar wie voranstehend definiert sind, und einer anschließenden reduktiven Aminierung der entsprechenden Oxoverbindungen. Die Oxoverbindungen werden in einer Eintopfreaktion direkt zu den entsprechenden Diarylaminen umgesetzt. Das erfindungsgemäße Verfahren umfasst also die Hydrocarbonylierung/Hydroformylierung durch Synthesegas, d.h. ein Gemisch aus Kohlenmonoxid (CO) und Wasserstoff (H₂) und die reduktive Aminierung mit Aminen der allgemeinen Formel HNR¹R², worin R¹ und R² wie voranstehend definiert sind. Die Hydroaminomethylierung führt in Gegenwart primärer Amine zu sekundären Aminen während die Umsetzung mit sekundären Aminkomponenten zu tertiären Aminen führt. Vorzugsweise wird bei dem erfindungsgemäßen Verfahren Diisopropylamin als Aminkomponente eingesetzt.

Gemäß einer Ausführungsform des erfindungsgemäßen Verfahrens steht der Substituent X für Hydroxy (OH). Dies erlaubt, ausgehend von 1,1-Diarylethenverbindungen, die Hydroformylierung zu Chromen/Lactol-Systemen gemäß dem nachstehend dargestellten allgemeinen Formelschema

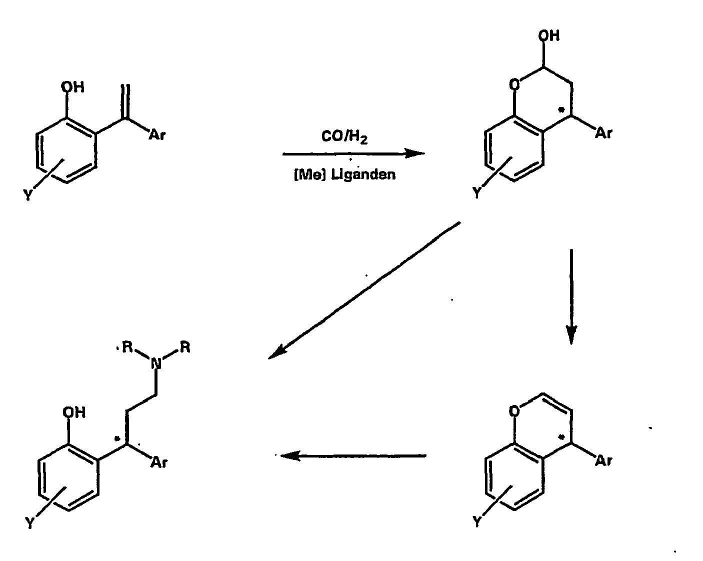

Diese Zwischenprodukte werden durch bekannte Reaktionen in Derivate des 2-[(3R)-3-(+)-(diisopropylamino)-1-phenylpropyl]-4-(hydroxymethyl)phenol, deren acylierte Verbindungen und Salze überführt. Eine genaue Beschreibung dieser Reaktionen sind in der EP 0 957 073 zusammengefasst.

Verfahrensvorschriften zur reduktiven Aminierung von Verbindungen des Lactoltyps finden sich auch in der WO 99/58478.

Die vorgenannten 1,1-Diarylethen-Verbindungen gemäß allgemeiner Formel II können nach an sich bekannten Verfahren hergestellt werden wie sie beipielsweise in Yamaguchi M; Arisawa M; Omata K; Kabuto K; Hirama M; Uchimaru T; Journal of Organic Chemistry 1998, 63(21), 7298-7305 und Yamaguchi M; Hayashi A; Hirama M; Journal of the American Chemical Society 1995, 117(3), 1151-2 beschrieben sind.

Das erfindungsgemäße Verfahren wird im allgemeinen bei einer Temperatur im Bereich von 50 bis 200°C und vorzugsweise 100 bis 140°C durchgeführt.

Der Reaktionsdruck beträgt dabei 40 bis 200 bar und vorzugsweise 80 bis 120 bar.

Bei der Hydroformylierung wird ein Gemisch aus Kohlenmonoxid und Wasserstoff (Synthesegas) verwendet, wobei das Verhältnis von Kohlenmonoxid (CO) zu Wasserstoff (H₂) im allgemeinen 10/90 bis 90/10 und vorzugsweise 70/30 bis 90/10 beträgt.

Die Breite der Druckverhältnisse ergibt sich aus der Notwendigkeit, dass die Verhältnisse von Ligand und Katalysator für jedes Substrat separat optimiert werden und daher abhängig sind von Substrat, Katalysatorvorstufe und Ligand etc.

Die Reaktionsdauer liegt im allgemeinen im Bereich von 2 Stunden bis 4 Tagen und vorzugsweise im Bereich von 1 bis 3 Tagen.

Die vorgenannten Reaktionszeiten werden von der jeweiligen Geräteausstattung beeinflusst. Bei einer optimalen Gaseinführung können auch kürzere Reaktionszeiten realisiert werden.

Der bei dem erfindungsgemäßen Verfahren verwendete Katalysator umfasst ein oder mehrere Übergangsmetalle ausgewählt aus der Gruppe bestehend aus Ruthenium, Rhodium, Platin, Cobalt, Iridium, Palladium, Nickel, wobei Rhodium bevorzugt ist.

In dem erfindungsgemäßen Verfahren wird der Katalysator in situ aus einem Katalysatorvorläufer und einem Liganden gebildet.

Geeignete Katalysatorvorläufer sind vorzugsweise [Rh(cod)Cl]₂ und/oder Rh(acac)(CO)₂ oder vergleichbare Rhodiumkomplexe.

Die Liganden sind nachfolgend zusammen mit der einschlägigen Abkürzung aufgelistet:
BINAPHOS = R-2-(Diphenylphosphino)-1,2'-binaphthalen-2'-yl-(S)-1,1'-binaphthalen-2,2'-diylphosphit,
DIOP = (2,2-Dimethyl-4,5-diphenylphosphinomethyl)-1,3-dioxolan,
DIOP-DBP = (2,2-Dimethyl-4,5-bis(5H-dibenzophosphol-5-ylmethyl)-1,3-dioxolan,
DPPB = 1,4-Bis(diphenylphosphino)butan,
CHIRAPHOS = 2,3-Bis(diphenylphosphino)butan,
CBDPP = 1,2-Bis(diphenylphosphinomethyl)cyclobutan,
CBDBP = 1,2-Bis(5H-dibenzophosphol-5-ylmethyl)cyclobutan,
CHDPP = 1,2-Bis(diphenylphosphinomethyl)cyclohexan,
CHDBP = 1,2-Bis(5H-dibenzophosphol-5-ylmethyl)cyclohexan,
CHDPPO = 1,2-Bis(diphenylphosphinoxy)cyclohexan,
BzMePhP* = Benzyl-methyl-phenylphosphin,
CAMP = Cyclohexyl-o-anisyl-methylphosphin,
NMDPP = Neomenthyldiphenylphosphin,
PAMP = Phenyl-o-anisyl-methylphosphin,
BPPM = (2S,4S)-N-tert.Butoxycarbonyl-4-diphenylphosphino-2-diphenylphosphinomethylpyrrolidin,
o-DPPB = ortho-Diphenylphosphanylbenzoyl,
PBu3 = Tributylphosphin,
BINAP = 2,2'-Bis-(diphenylphosphino)-1,1'-binaphthyl.

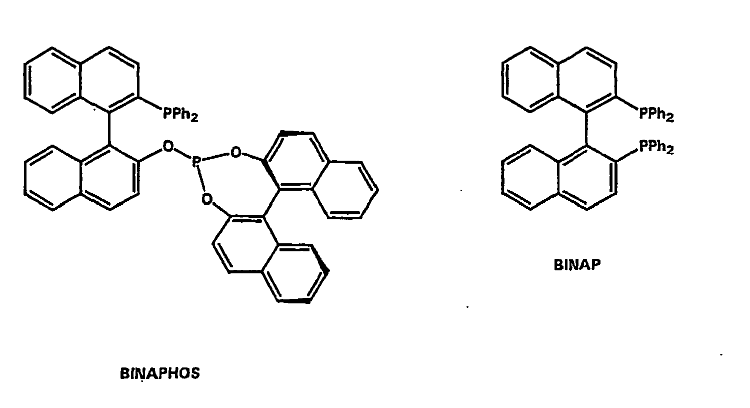

Bevorzugte Liganden sind Tributylphosphin, (+)- oder (-)-(2,2-Dimethyl-4,5-diphenylphosphinomethyl)-1,3-dioxolan, (R)-oder (S)-BINAP und/oder (R,S)-BINAPHOS.

Wird als Ligand Tributylphosphin verwendet, so beträgt das Verhältnis von Ligand zu Rhodium im allgemeinen 1:1 bis 25:1 und vorzugsweise 4:1 bis 10:1.

Bei Verwendung von (R)- oder (S)-BINAP beträgt es 1:1 bis 6:1 und vorzugsweise 1:1 bis 2:1.

Bei (R,S)-BINAPHOS schließlich beträgt es 1:1 bis 6:1 und vorzugsweise 1:1 bis 2:1.

Das erfindungsgemäße Verfahren beruht, wie bereits voranstehend ausgeführt, auf der Hydroaminomethylierung von 1,1-Diarylethenen unter Verwendung eines geeigneten Katalysatorsystems. Es weist den Vorteil auf, daß es als Eintopfreaktion durchgeführt wird, was die direkte Isolierung der gewünschten Diarylpropylaminderivate ermöglicht.

Das Chiralitätszentrum in Position 3 der 3,3-Diarylpropylaminderivate läßt sich durch geeignete Wahl von chiralen Liganden für das metallische Katalysatorzentrum in stereoselektiver Weise erzeugen (Ligandensteuerung). Ist eine der Arylgruppen in dem als Ausgangmaterial verwendeten 1,1-Diarylethen in ortho-Position durch ein Heteroatom substituiert, welches mit chiralen Gruppen modifiziert ist, wird die chirale Synthese durch das Substrat gesteuert (Substratsteuerung). Dies lässt sich mit Homoallylalkoholen vergleichen, deren Konformation durch den planaren Aromaten vorfixiert ist. Schließlich lässt die Kombination dieser Methoden (Liganden- und Substratsteuerung) die doppelte Stereoseitendifferenzierung zu.

Das erfindungsgemäße Verfahren wird durch die folgenden Beispiele näher erläutert. Hierin werden die folgenden Definitionen verwendet:
- DC: Dünnschichtchromatographie
- HPLC: High Performance Liquid Chromatography
- NMR: kernmagnetische Resonanz
- °C: Grad Celsius
- RT: Raumtemperatur
- THF: Tetrahydrofuran
- acac: Acetonylacetat
- cod: cis, cis-1,5-Cyclooctadien
- abs.: absolut(em)
- MTBE: Methyl-tert.Butylether
- h: Stunde(n)
- TMS: Tetramethylsilan

### Geräteliste

Die NMR-Spektren wurden mit einem DRX 400 der Firma Bruker aufgenommen. TMS wurde als interner Standard eingesetzt.

Massenspektren wurden auf einem Finnigan CA 5 gemessen. Die Elementarzusammensetzung wurde mit einem Leco CHNS-932 bestimmt.

Gaschromatographische Untersuchungen wurden mit einem CarloErba GC-4160 mit 25 m oder mit einem Fisous GC-8130 mit 30 m CP sil-5 Kapillaren durchgeführt.

### Beispiel 1: Herstellung von 2-Methoxy-5-methyl-benzophenon

Ansatz:
5.00 g (23.5 mmol) 2-Hydroxy-5-methyl-benzophenon
6.67 g (47.1 mmol) Methyliodid
4.50 g (32.6 mmol) Kaliumcarbonat
50 ml abs. Aceton

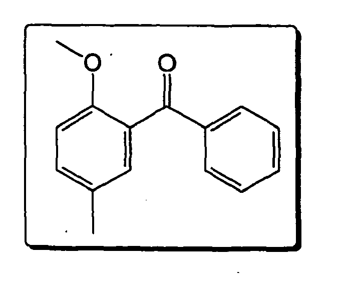

Durchführung:
Zu einer Lösung aus 5.00 g (23.5 mmol) 2-Hydroxy-5-methyl-benzophenon und 6.67 g (47.1 mmol) Methyliodid in 50 ml abs. Aceton werden 4.50 g (32.6 mmol) Kaliumcarbonat gegeben. Das Reaktionsgemisch erhitzt man anschließend 5 h unter Rückfluss. Nach Abkühlen werden zu der Suspension 50 ml Wasser und 50 ml Petrolether (30/60) gegeben. Man trennt die organische Phase ab, extrahiert die wässrige Phase zweimal mit 75 ml Petrolether (30/60) und wäscht die vereinigten organischen Phasen zweimal mit 50 ml 10 %iger NaOH-Lösung. Die organische Phase wird anschließend über Magnesiumsulfat getrocknet und das Lösungsmittel wird entfernt. Man erhält 3.80 g (16.8 mmol, 71 %) 2-Methoxy-5-methyl-benzophenon.

Ausbeute: 3.80 g (16.8 mmol, 71 %) 2-Methoxy-5-methyl-benzophenon
¹H-NMR (400 MHz, CDCl₃): δ [ppm] = 2.21 (s, 3H, CH₃), 3.56 (s, 3H, OCH₃), 6.77 (d, ³J = 8.4 Hz, 1H, PhH), 7.06 (d, ⁴J = 1.9 Hz, 1H, PhH), 7.15 (m, 1H, PhH), 7.31 (t, ³J = 7.7 Hz, 2H, PhH), 7.43 (m, 1H, PhH), 7.71 (dd, ³J = 8.3 Hz, ²J = 1.2 Hz, 2H, PhH).
¹³C-NMR (100 MHz, CDCl₃): δ [ppm] = 20.3 (CH₃), 55.6 (OCH₃), 111.4 (CHₐᵣₒₘ), 128.1 (2 x CHₐᵣₒₘ), 128.5 (Cqₐᵣₒₘ), 129.7 (2 x CHₐᵣₒₘ), 129.8 (Cqₐᵣₒₘ), 129.9 (CHₐᵣₒₘ), 132.2 (CHₐᵣₒₘ), 132.8 (CHₐᵣₒₘ), 137.8 (Cqₐᵣₒₘ), 155.2 (Cqₐᵣₒₘ), 196.6 (C=O).

### Beispiel 2: Herstellung von 2-Benzyloxy-5-methyl-benzophenon

Ansatz:
2.00 g (9.4 mmol) 2-Hydroxy-5-methyl-benzophenon
1.66 g (9.7 mmol) Benzylbromid
1.73 g (12.5 mmol) Kaliumcarbonat
12 ml abs. Aceton

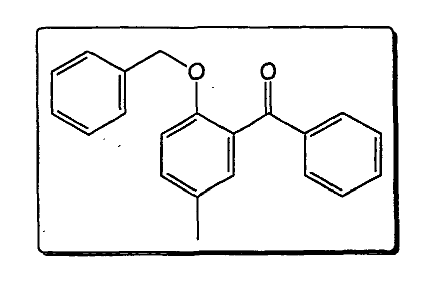

Durchführung:
Zu einer Lösung aus 2.00 g (9.4 mmol) 2-Hydroxy-5-methyl-benzophenon und
1.66 g (9.7 mmol) Benzylbromid in 12 ml abs. Aceton werden 1.73 g (12.5 mmol) Kaliumcarbonat gegeben. Das Reaktionsgemisch erhitzt man anschließend 16 h unter Rückfluss. Nach Abkühlen der Suspension wird diese filtriert und der Rückstand mit 50 ml Diethylether gewaschen. Nach Entfernen des Lösungsmittels am Rotationsverdampfer erhält man 2.74 g (9.1 mmol, 96 %) 2-Benzyloxy-5-methyl-benzophenon.

Ausbeute: 2.74 g (9.1 mmol, 96 %) 2-Benzyloxy-5-methyl-benzophenon
¹H-NMR (400 MHz, CDCl₃): δ [ppm] = 2.31 (s, 3H, CH₃), 4.94 (s, 2H, OCH₂), 6.92 (m, 3H, PhH), 7.17 (m, 3H, PhH), 7.23 (m, 2H, PhH), 7.41 (t, ³J = 7.6 Hz, 2H, PhH), 7.53 (m, 1H, PhH), 7.81 (m, 2H, PhH).
¹³C-NMR (100 MHz, CDCl₃): δ [ppm] = 20.3 (CH₃), 70.1 (OCH₂), 112.8 (CHₐᵣₒₘ), 126.5 (2 x CHₐᵣₒₘ), 127.4 (CHₐᵣₒₘ), 128.2 (4 x CHₐᵣₒₘ), 129.1 (Cqₐᵣₒₘ), 129.6 (2 x CHₐᵣₒₘ), 130.1 (CHₐᵣₒₘ), 130.4 (Cqₐᵣₒₘ), 132.4 (CHₐᵣₒₘ), 132.6 (CHₐᵣₒₘ), 136.5 (Cqₐᵣₒₘ), 138.3 (Cqₐᵣₒₘ), 154.3 (Cqₐᵣₒₘ), 196.9 (C=O).

Schmelzpunkt: 78°C

| Elementaranalyse: | ber.: | C 83.4 | H 6.0 |
|---|---|---|---|
| | gef.: | C 83.2 | H 5.8 |

### Beispiel 3: Herstellung von 1-Methoxy-4-methyl-2-(1-phenylvinyl)-benzol

Ansatz:
3.70 g (16.3 mmol) 2-Methoxy-5-methyl-benzophenon
7.58 g (21.2 mmol) Methyltriphenylphosphonium-bromid
17.0 ml (21.2 mmol) n-Butyllithium (1.25M in *n*-Hexan)
20 ml abs. THF

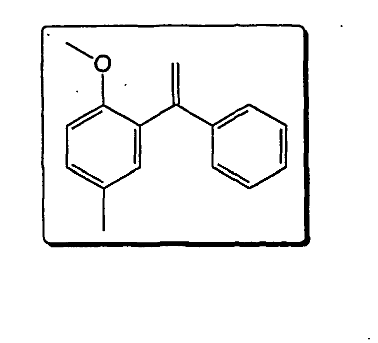

Durchführung:
Zu einer Suspension aus 7.58 g (21.2 mmol) Methyltriphenylphosphoniumbromid in 15 ml abs. THF werden bei Raumtemperatur innerhalb von 10 min 17.0 ml (21.2) n-Butyllithium (1.25M in n-Hexan) zugetropft. Die erhaltene rötliche Lösung kühlt man auf -78 °C und gibt anschließend innerhalb von 15 min eine Lösung aus 3.70 g (16.3 mmol) 2-Methoxy-5-methyl-benzophenon in 5 ml abs. THF hinzu. Es wird zunächst 20 min bei -78 °C und weitere 8 h bei Raumtemperatur gerührt. Anschließend gibt man zu dem Reaktionsgemisch 80 ml Wasser und extrahiert zweimal mit je 50 ml Diethylether. Die vereinigten organischen Phasen werden über Magnesiumsulfat getrocknet und das Lösungsmittel wird entfernt. Die Reinigung des erhaltenen Rohproduktes erfolgt säulenchromatographisch an Kieselgel mit Toluol als Laufmittel. Man erhält 3.03 g (13.5 mmol, 83 %) 1-Methoxy-4-methyl-2-(1-phenylvinyl)-benzol.

Ausbeute: 3.03 g (13.5 mmol, 83 %) 1-Methoxy-4-methyl-2-(1-phenylvinyl)-benzol
¹H-NMR (400 MHz, CDCl₃): δ [ppm] = 2.22 (s, 3H, CH₃), 3.51 (s, 3H, OCH₃), 5.22 (s, 1H, =CH₂), 5.63 (s, 1H, =CH₂), 6.71 (d, ³J = 8.3 Hz, 1H, PhH), 6.97 (s, 1H, PhH), 7.01 (d, ³J = 8.0 Hz, 1H, PhH), 7.16 (m, 5H, PhH).
¹³C-NMR (100 MHz, CDCl₃): δ [ppm] = 20.4 (CH₃) , 55.8 (OCH₃), 111.3 (CHₐᵣₒₘ), 115.2 (=CH₂), 126.3 (2 x CHₐᵣₒₘ), 127.2 (CHₐᵣₒₘ), 128.0 (2 x CHₐᵣₒₘ), 129.2 (CHₐᵣₒₘ), 129.8 (Cqₐᵣₒₘ), 130.8 (Cqₐᵣₒₘ), 131.8 (CHₐᵣₒₘ), 141.0 (Cqₐᵣₒₘ), 147.0 (Cq_{olefin}), 155.0 (Cqₐᵣₒₘ).

| Elementaranalyse: | ber.: | C 85.7 | H 7.2 |
|---|---|---|---|
| | gef.: | C 85.7 | H 7.2 |

### Beispiel 4: Herstellung von 1-Benzyloxy-4-methyl-2 -(1-phenylvinyl)-benzol

Ansatz:
1.41 g (4.7 mmol) 2-Benzyloxy-5-methyl-benzophenon
2.16 g (6.1 mmol) Methyltriphenylphosphoniumbromid
4.9 ml (6.i mmol) n-Butyllithium (1.25M in n-Hexan)
17 ml abs. THF

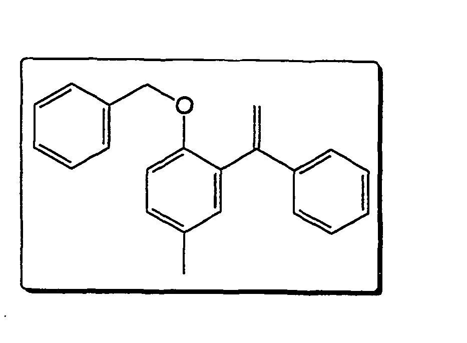

Durchführung:
analog Beispiel 3

Ausbeute: 1.23 g (4.1 mmol, 88 %) 1-Benzyloxy-4-methyl-2-(1-phenylvinyl)-benzol
¹H-NMR (400 MHz, CDCl₃): δ [ppm] = 2.32 (s, 3H, CH₃), 4.84 (s, 2H, OCH₂), 5.32 (d, ³J = 1.4 Hz, 1H, =CH₂), 5.65 (d, ³J = 1.4 Hz, 1H, =CH₂), 6.83 (m, 3H, PhH), 7.07 -7.37 (m, 10H, PhH).
¹³C-NMR (100 MHz, CDCl₃): δ [ppm] = 20.5 (CH₃), 70.1 (OCH₂), 112.5 (CHₐᵣₒₘ), 115.5 (=CH₂), 126.5 (2 x CHₐᵣₒₘ), 126.8 (2 x CHₐᵣₒₘ), 127.2 (CHₐᵣₒₘ), 127.3 (CHₐᵣₒₘ), 128.1 (4 x CHₐᵣₒₘ), 129.3 (CHₐᵣₒₘ), 130.1 (Cqₐᵣₒₘ), 131.3 (Cqₐᵣₒₘ), 132.1 (CHₐᵣₒₘ), 137.1 (Cqₐᵣₒₘ), 141.6 (Cqₐᵣₒₘ), 147. 6 (Cq_{olefin}), 153. 9 (Cqₐᵣₒₘ).
MS (EI, 70 eV) : m/z [%] = 300 (39, M⁺), 285 (11), 262 (6), 251 (9), 209 (100), 200 (9), 195 (20), 181 (20), 165 (19), 115 (3), 91 (95), 65 (9).

| Elementaranalyse: | ber.: | C 88.0 | H 6.7 |
|---|---|---|---|
| | gef.: | C 87.6 | H 6.7 |

### Beispiel 5: Herstellung von Diisopropyl-[3-(2-methoxy-5-methylphenyl)-3-phenylpropyl]-amin

Ansatz:
0.700 g (3.1 mmol) 1-Methoxy-4-methyl-2-(1-phenyl-vinyl)-benzol
0.354 g (3.5 mmol) Diisopropylamin
s. Tab. 1 Katalysatorvorläufer
s. Tab. 1 Tributylphosphin
10 ml abs. Dioxan

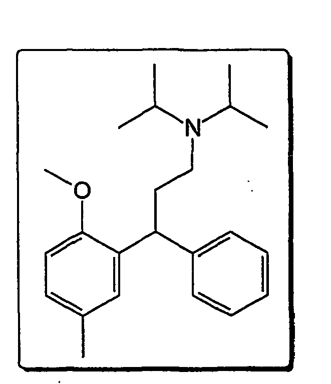

Durchführung:
0.700 g (3.1 mmol) 1-Methoxy-4-methyl-2-(1-phenylvinyl)-benzol, 0.354 g (3.5 mmol) Diisopropylamin, eine definierte Menge Katalysatorvorläufer (s. Tab. 1), eine definierte Menge Tributylphosphin (s. Tab. 1) und 10 ml abs. Dioxan werden in einem Druckbehälter in einer CO/H₂-Atmosphäre unter den in Tab. 1 angegebenen Bedingungen gerührt. Nach Abkühlen und Entspannen des Druckbehälters wird die Reaktionslösung über Aluminiumoxid (Aktivität II - III, basisch) absorptiv filtriert (Elutionsmittel: MTBE). Nach Entfernen des Lösungsmittels erfolgt die Reinigung des Rohproduktes säulenchromatographisch an Aluminiumoxid (Aktivität I, neutral) mit Petrolether (30/60) / MTBE = 5/1 (v/v) als Elutionsmittel.

**Tabelle 1: Ergebnisse der Hydroaminomethylierung mit 1-Methoxy-4-methyl-2-(1-phenylvinyl)-benzol**

| Nr. | PBu₃/Rh | T [°C] | p (CO/H₂) [bar] | t [d] | hydr.Edu kt [%]* | Produkt [%]* |
|---|---|---|---|---|---|---|
| 1** | 25/1 | 115 | 80/20 | 4 | - | - |
| 2** | 6/1 | 135 | 90/10 | 4 | ca. 1 | 38 (22) |
| 3** | 6/1 | 140 | 90/10 | 5 | 11 | 49 (37) |
| 4*** | 16/1 | 110 | 90/10 | 3 | ca. 2 | 12 |
| 5*** | 8/1 | 130 | 90/10 | 3 | 6 | 85 |
| 6*** | - | 130 | 90/10 | 3 | 11 | 70 |

| | | | | | | |
|---|---|---|---|---|---|---|
| * : GC-Anteile (Werte in Klammern entsprechen isolierten Ausbeuten) **: [Rh(cod)Cl]₂ (0.5 mol-% bezogen auf Olefin) *** : Rh(acac)(CO)₂ (1 mol-% bezogen auf Olefin) | | | | | | |

¹H-NMR (400 MHz, CDCl₃): δ [ppm] = 0.92 (d, ³J = 6.5 Hz, 6H, 2 x CHC*H₃*), 0.92 (d, ³J = 6.5 Hz, 6H, 2 x CHC*H₃*), 2.12 (m, 2H, CHC*H₂*), 2.25 (s, 3H, PhCH₃), 2.33 (m, 2H, NCH₂), 2.96 (sept, ³J = 6.5 Hz, 2H, 2 x CHMe₂), 3.72 (s, 3H, OCH₃), 4.34 (t, ³J = 7.6 Hz, 1H, CHPh₂), 6.69 (d, ³J = 8.3 Hz, 1H, PhH), 6.92 (dd, ³J = 8.1 Hz, ⁴J = 1. 9 Hz, 1H, PhH), 7.05 (d, ⁴J = 1.9 Hz, 1H, PhH), 7.12 (t, ³J = 7.0 Hz, 1H, PhH), 7.13 - 7.24 (m, 4H, PhH).
¹³C-NMR (100 MHz, CDCl₃): δ [ppm] = 20.5 (2 x CHC*H₃*), 20.7 (2 x CHC*H₃*), 20.7 (PhCH₃), 37.0 (CHCH₂), 41.3 (PhCH), 44.1 (NCH₂), 48.7 (2 X NCH), 55.5 (OCH₃), 110.6 (CHₐᵣₒₘ), 125.6 (CHₐᵣₒₘ), 127.1 (CHₐᵣₒₘ), 128.0 (2 x CHₐᵣₒₘ), 128.2 (2 x CHₐᵣₒₘ), 128.3 (CHₐᵣₒₘ), 129.5 (Cqₐᵣₒₘ), 133.4 (Cqₐᵣₒₘ), 145.1 (Cqₐᵣₒₘ), 154.9 (Cqₐᵣₒₘ).
GC-MS (EI, 70EV): m/z [%] = 340 (M⁺+1, 45), 324 (6), 296 (1), 211 (3), 126 (3), 114 (100), 100 (20), 91 (7), 72 (13).

| Elementaranalyse: | C₂₃H₃₃NO (339.26) | | | |
|---|---|---|---|---|
| | ber.: | C 81.4 | H 9.8 | N 4.1 |
| | gef.: | C 80.9 | H 9.4 | N 4.3 |

### Beispiel 6: Herstellung von [3-(2-Benzyloxy-5-methylphenyl)-3-phenylpropyl]-diisopropylamin

Ansatz:
0.601 g (2.00 mmol) 1-Benzyloxy-4-methyl-2-(1-phenylvinyl)-benzol
0.229 g (2.26 mmol) Diisopropylamin
5.2 mg (0.02 mmol) Rh(acac)(CO)₂ s. Tab. 2 Tributylphosphin 10 ml abs. Dioxan'

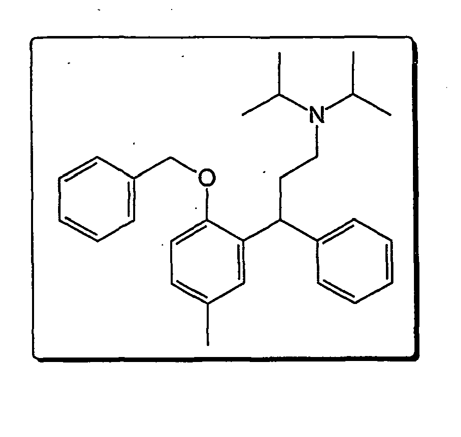

Durchführung:
analog Beispiel 5

**Tabelle 2: Ergebnisse der Hydroaminomethylierung mit 1-Benzyloxy-4-methyl-2-(1-phenylvinyl)-benzol**

| Nr. | PBu₃/Rh | T [°C] | p (CO/H₂) [bar] | t [d] | Produkt [%]* |
|---|---|---|---|---|---|
| 7** | 8/1 | 130 | 90/10 | 3 | 84 (79) |
| 8** | 2/1 | 100 | 90/10 | 3 | 4 |
| 9** | - | 130 | 90/10 | 1 | 49 |
| 10** | - | 130 | 90/10 | 3 | 76 |

| | | | | | |
|---|---|---|---|---|---|
| * : GC-Anteile (Werte in Klammern entspricht isolierter Ausbeute) **: Rh(acac)(CO)₂ (1 mol-% bezogen auf Olefin) | | | | | |

¹H-NMR (400 MHz, CDCl₃): δ [ppm] = 0.90 (d, ³J = 6.5 Hz, 12H, 4 x CHC*H₃*), 2.13 (m, 2H, CHC*H₂*), 2.26 (s, 3H, PhCH₃), 2.33 (m, 2H, NCH₂), 2.94 (sept, ³J = 6.5 Hz, 2H,
2 x CHMe₂), 4.39 (t, ³J = 7.7 Hz, 1H, CHPh₂), 4.94 (d, ²J = 15.5 Hz , 1H, OCH₂), 4.97 (d, ²J = 15.5 Hz , 1H, OCH₂), 6.73 (d, ³J = 8.2 Hz, 1H, PhH), 6.91 (dd, ³J = 8.2 Hz, ⁴J = 1.7 Hz, 1H, PhH), 7.10 - 7.36 (m, 11H, PhH).
¹³C-NMR (100 MHz, CDCl₃) : δ [ppm] = 20.5 (2 x CHC*H*₃), 20.6 (2 x CHC*H*₃), 20.7 (PhCH₃), 37.0 (CH*C*H₂), 41.5 (PhCH), 44.1 (NCH₂), 48.8 (2 X NCH), 70.1 (OCH₂), 111.7 (CHₐᵣₒₘ), 125.6 (CHₐᵣₒₘ), 127.1 (CHₐᵣₒₘ), 127.3 (2 x CHₐᵣₒₘ), 127.6 (CHₐᵣₒₘ), 128.0 (2 x CHₐᵣₒₘ), 128.3 (2 x CHₐᵣₒₘ), 128.3 (2 x Chₐᵣₒₘ), 128.4 (CHₐᵣₒₘ), 129.8 (Cqₐᵣₒₘ), 133.6 (Cqₐᵣₒₘ), 137.5 (Cqₐᵣₒₘ), 145.1 (Cqₐᵣₒₘ), 153.9 (Cqₐᵣₒₘ).
MS (EI, 70EV): m/z [%] = 415 (M⁺, 23), 400 (25), 339 (4), 324 (6), 287 (1), 223 (3), 197 (3), 165 (1), 114 (100), 99 (4), 91 (18), 72 (11).

| Elementaranalyse: | (C₂₉H₃₇NO, M = 415.29) | | | |
|---|---|---|---|---|
| | ber.: | C 83.8 | H 9.0 | N 3.4 |
| | gef.: | C 84.0 | H 9.0 | N 3.4 |

### Beispiel 7: Herstellung von 4-Hydroxy-3-(1-phenylvinyl)-benzoesäureethylester

Ansatz:
4.98 g (30 mmol) p-Hydroxybenzoesäureethylester
3.06 g (30 mmol) Phenylacetylen
31.26 g (120 mmol) Zinntetrachlorid
22.24 g (120 mmol) Tributylamin
150 ml 1,2-Dichlorethan

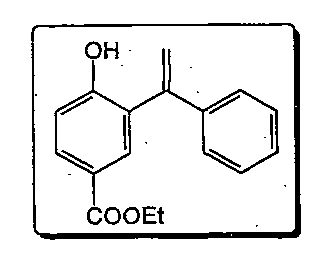

Durchführung:
Unter einer Argonatmosphäre wird eine Lösung aus 4.98 g (30 mmol) p-Hydroxy-benzoesäureethylester, 3.06 g (30 mmol) Phenylacetylen, 31.26 g (120 mmol) Zinntetrachlorid und 22.24 (120 mmol) Tributylamin in 150 ml 1,2-Dichlorethan 1 h unter Rückfluss erhitzt. Anschließend gibt man zu dem Reaktionsgemisch 60 ml 4 M KOH und 30 ml Ethanol und erhitzt 1h unter Rückfluss. Nach Abkühlen wird die Lösung mit 4 M HCl angesäuert und zweimal mit je 150 ml Diethylether extrahiert. Die vereinigten organischen Phasen trocknet man über MgSO₄ und entfernt das Lösungsmittel am Rotationsverdampfer. Der verbleibende Rückstand wird absorptiv filtriert (Kieselgel, MTBE) und das so erhaltene Rohprodukt säulenchromatographisch gereinigt (Kieselgel, Toluol/MTBE = 8/1 (v/v)). Man erhält 2.63 g (9.8 mmol, 33 %) 4-Hydroxy-3-(1-phenyl-vinyl)-benzoesäure-ethylester.

Ausbeute: 2.63 g (9.8 mmol, 33 %) 4-Hydroxy-3-(1-phenyl-vinyl)-benzoesäure-ethylester.
¹H-NMR (400 MHz, CDCl₃): δ [ppm] = 1.36 (t, ³J = 7.1 Hz, 3H, CH₃), 4.33 ( q, ³J = 7.1 Hz, 2H, OCH₂), 5.44 (d, ²J = 0.9 Hz, 1H, =CH₂), 5.91 (d, ²J = 0.9 Hz, 1H, =CH₂), 6.97 (d, ³J = 8.5 Hz, 1H, PhH), 7.34 (m, 5H, PhH), 7.89 (d, ⁴J = 2.1 Hz, 1H, PhH), 7.96 (dd, ³J = 8.5 Hz, ⁴J = 2.1 Hz, 1H, PhH).
¹³C-NMR (100 MHz, CDCl₃): δ [ppm] = 14.3 (CH₃), 60.8 (OCH₂), 115.8 (CHₐᵣₒₘ), 117.5 (=CH₂), 122.9 (Cqₐᵣₒₘ), 126.9 (2 x CHₐᵣₒₘ), 127.5 (Cqₐᵣₒₘ), 128.8 (3 x CHₐᵣₒₘ), 131.3 (CHₐᵣₒₘ), 132.3 (CHₐᵣₒₘ), 138.7 (Cqₐᵣₒₘ), 144.3 (Cq_{olefin}), 157.1 (Cqₐᵣₒₘ), 166.3 (COOEt).
MS (EI, 70EV): m/z [%] = 268 (M⁺, 98), 267 (100), 253 (35), 239 (32), 225 (13), 223 (24), 194 (6), 165 (20), 152 (12), 115 (5), 111 (6), 104 (7).

### Beispiel 8: Herstellung von 4-Benzyloxy-3-(1-phenylvinyl)-benzoesäureethylester-

Ansatz:
0.990 g (3.7 mmol) 4-Hydroxy-3-(1-phenylvinyl)-benzoesäureethylester
0.652 g (3.8 mmol) Benzylbromid
0.680 g (4.9 mmol) Kaliumcarbonat
7 ml abs. Aceton

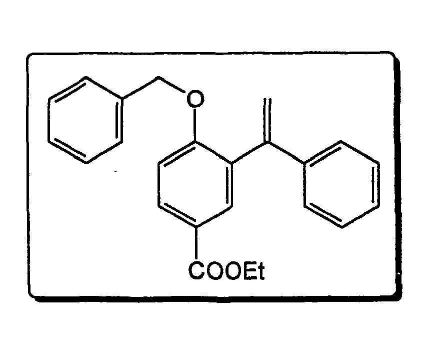

Durchführung:
Zu einer Lösung aus 0.990 g (3.7 mmol) 4-Hydroxy-3-(1-phenylvinyl)-benzoesäureethylester und 0.652 g (3.8 mmol) Benzylbromid in 7 ml abs. Aceton werden 0.680 g (4.9 mmol) Kaliumcarbonat gegeben. Das Reaktionsgemisch erhitzt man anschließend 4 h unter Rückfluss. Nach Abkühlen der Suspension wird diese filtriert und der Rückstand mit 50 ml Diethylether gewaschen. Nach Entfernen des Lösungsmittels am Rotationsverdampfer erhält man 1.280 g (3.6 mmol, 97 %) 4-Benzyloxy-3-(1-phenylvinyl)-benzoesäureethylester als farbloses Öl.

Ausbeute: 1.280 g (3.6 mmol, 97 %) 4-Benzyloxy-3-(1-phenylvinyl)-benzoesäureethylester.
¹H-NMR (400 MHz, CDCl₃): δ [ppm] = 1.38 (t, ³J = 7.1 Hz, 3H, CH₃), 4.36 (q, ³J = 7.1 Hz, 2H, OC*H₂*CH₃), 4.93 (s, 2H, OCH₂Ph), 5.36 (d, ²J = 1.2 Hz, 1H, =CH₂), 5.70 (d, ²J = 1.2 Hz, 1H, =CH₂), 6.80 (dd, ³J = 7.6 Hz, ⁴J = 1.6 Hz, 2H, PhH), 6.94 (d, ³J = 9.3 Hz, 1H, PhH), 7.18 (m, 3H, PhH), 7.29 (m, 5H, PhH), 8.03 (m, 2H, PhH).
¹³C-NMR (100 MHz, CDCl₃): δ [ppm] = 14.4 (CH₃), 60.7 (OCH₂CH₃), 69.9 (OCH₂Ph), 111.4 (CHₐᵣₒₘ), 116.3 (=CH₂), 123.0 (Cqₐᵣₒₘ), 126.4 (2 x CHₐᵣₒₘ), 126.7 (2 x CHₐᵣₒₘ), 127.4 (CHₐᵣₒₘ), 127.6 (CHₐᵣₒₘ), 128.2 (4 x CHₐᵣₒₘ), 131.2 (CHₐᵣₒₘ), 131.3 (Cqₐᵣₒₘ), 132.8 (CHₐᵣₒₘ), 136.0 (Cqₐᵣₒₘ), 141.0 (Cqₐᵣₒₘ), 146.8 (Cq_{olefin}), 159.6 (Cqₐᵣₒₘ), 166.3 (COOEt).
MS (EI, 70 eV): m/z [%] = 358 (37, M⁺), 343 (9), 329 (6), 313 (10), 285 (13), 267 (95), 253 (11), 239 (6), 207 (3), 194 (12), 165 (14), 139 (2), 105 (3), 91 (100), 65 (10).

### Beispiel 9: Herstellung von 4-Benzyloxy-3-(3-diisopropylamino-1-phenyl-propyl)-benzoesäureethylester

Ansatz:
1.222 g (3.41 mmol) 4-Benzyloxy-3-(1-phenylvinyl)-benzoesäureethylester
0.390 g (3.85 mmol) Diisopropylamin
8.8 mg (0.03 mmol) Rh(acac)(CO)₂
68 µl (0.27 mmol) Tributylphosphin 15 ml abs. Dioxan

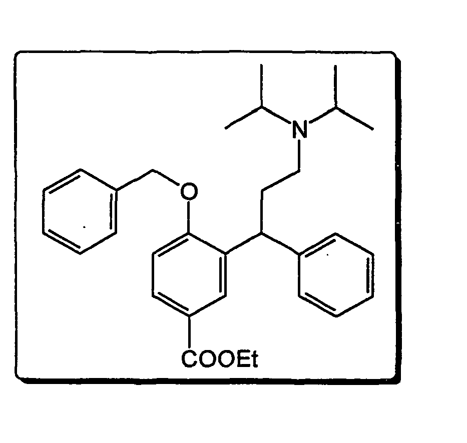

Durchführung:
analog Beispiel 5 mit p(CO/H₂) = 90/10 bar, 130°C, 65 h. Die Reinigung des Rohproduktes erfolgt säulenchromatographisch (Kieselgel, Petrolether/MTBE/NEt₃ (Lösungsmittelgemisch mit steigender Polarität; PE/MTBE = 1/1 → PE/MTBE/NEt₃ = 5/5/1).

Ausbeute: 1.155 g (2.4 mmol, 72 %) 4-Benzyloxy-3-(3-diisopropylamino-1-phenyl-propyl)-benzoesäureethylester.
¹H-NMR (400 MHz, CDCl₃): δ [ppm] = 0.90 (*t, ³J = 6.3 Hz, 12H, 4 x CHC*H*₃), 1.37 (t, ³J = 7.1 Hz, 3H, CH₂C*H*₃), 2.17 (m, 2H, CHC*H*₂), 2.33 (m, 2H, NCH₂), 2.95 (sept, ³J = 6.3 Hz, 2H, 2 x CHMe₂), 4.36 (m, 3H, CHPh₂, C*H*₂CH₃), 5.02 (d, ²J = 15.3 Hz, 1H, OCH₂Ph), 5.05 (d, ²J = 15.3 Hz, 1H, OCH₂Ph), 6.85 (d, ³J = 8.6 Hz, 1H, PhH), 7.16 (m, 1H, PhH), 7.20 - 7.25 (m, 6H, PhH), 7.32 (m, 3H, PhH), 7.87 (dd, ³J = 8.6 Hz, ⁴J = 2.1 Hz, 1H, PhH), 8.09 (d, ⁴J = 2.1 Hz, 1H, PhH).
¹³C-NMR (100 MHz, CDCl₃): δ [ppm] = 14.4 (CH₂CH₃) 20.5 (2 x CHCH₃), 20.6 (2 x CHCH₃), 36.6 (CHCH₂), 41.7 (PhCH), 43.9 (NCH₂), 48.6 (2 X NCH) , 60.6 (CH₂CH₃), 70.0 (OCH₂Ph), 111.0 (CHₐᵣₒₘ), 122.7 (Cqₐᵣₒₘ), 125.8 (CHₐᵣₒₘ), 127.4 (2 x CHₐᵣₒₘ), 127.9 (CHₐᵣₒₘ), 128.0 (2 x CHₐᵣₒₘ), 128.2 (2 x CHₐᵣₒₘ), 128.4 (2 X CHₐᵣₒₘ), 129.1 (CHₐᵣₒₘ), 129.3 (CHₐᵣₒₘ), 133.5 (Cqₐᵣₒₘ), 136.3 (Cqₐᵣₒₘ), 144.5 (Cqₐᵣₒₘ), 159.7 (Cqₐᵣₒₘ), 166.6 (COOEt).
MS (EI, 70EV) : m/z [%] = 473 (M⁺, 15), 458 (23), 428 (2), 360 (3) , 345 (2), 267 (4), 165 (2), 114 (100), 91 (41), 72 (90).

### Beispiel 10: Herstellung von 4-Hydroxy-3-(1-phenylvinyl)-benzoesäuremethylester

Ansatz:
4.56 g (30 mmol) p-Hydroxybenzoesäuremethylester
6.13 g (60 mmol) Phenylacetylen
31.26 g (120 mmol) Zinntetrachlorid
22.24 g (120 mmol) Tributylamin
150 ml 1,2-Dichlorethan

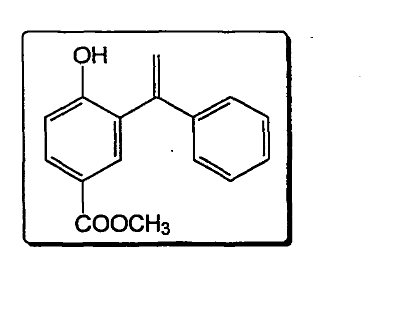

Durchführung: analog Beispiel 7

Ausbeute: 2.67 g (10.5 mmol, 35 %) 4-Hydroxy-3-(1-phenylvinyl)-benzoesäure-methylester.
¹H-NMR (400 MHz, CDCl₃) : δ [ppm] = 3.84 (s, 3H, CH₃), 5.42 (d, ²J = 1.0 Hz, 1H, =CH₂), 5.88 (d, ²J = 1.0 Hz, 1H, =CH₂), 6.97 (d, ³J = 8.5 Hz, 1H, PhH), 7.32 (m, 5H, PhH), 7.88 (d, ⁴J = 2.3 Hz, 1H, PhH), 7.94 (dd, ³J = 8.5 Hz, ⁴J = 2.3 Hz, 1H, PhH).
¹³C-NMR (100 MHz, CDCl₃): δ [ppm] = 51.9 (CH₃), 115.8 (CHₐᵣₒₘ), 117.4 (=CH₂), 122.4 (Cqₐᵣₒₘ), 126.8 (2 x CHₐᵣₒₘ), 127.6 (Cqₐᵣₒₘ), 128.7 (CHₐᵣₒₘ), 128.7 (2 x CHₐᵣₒₘ), 131.3 (CHₐᵣₒₘ), 132.4 (CHₐᵣₒₘ), 138.8 (Cqₐᵣₒₘ), 144.3 (Cq_{olefin}), 157.3 (Cqₐᵣₒₘ), 166.8 (COOMe).
MS (EI, 70EV) : m/z [%] = 254 (M⁺, 5), 253 (7), 239 (3) , 223 (1), 165 (1), 152 (1), 131 (1), 120 (22), 105 (44), 91 (100), 77 (37), 65 (13), 59 (16), 51 (20), 45 (17).

### Beispiel 11: Herstellung von 4-Benzyloxy-3-(1-phenyl-vinyl)-benzoesäuremethylester

Ansatz:
1.50 g (5.9 mmol) 4-Hydroxy-3-(1-phenyl-vinyl)-benzoesäuremethylester
1.04 g (6.1 mmol) Benzylbromid
1.24 g (8.9 mmol) Kaliumcarbonat
10 ml abs. Aceton

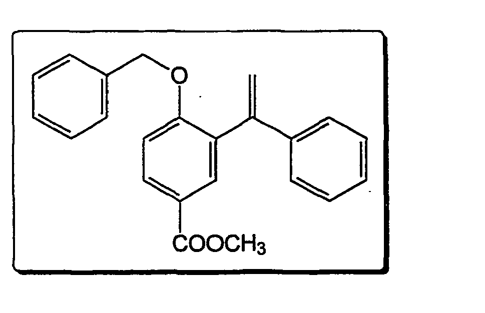

Durchführung:
Zu einer Lösung aus 1.50 g (5.9 mmol) 4-Hydroxy-3-(1-phenylvinyl)-benzoe-säuremethylester und 1.04 g (6.1 mmol) Benzylbromid in 10 ml abs. Aceton werden 1.24 g (8.9 mmol) Kaliumcarbonat gegeben. Das Reaktionsgemisch erhitzt man anschließend 4 h unter Rückfluss. Nach Abkühlen der Suspension wird diese filtriert und der Rückstand mit 50 ml Diethylether gewaschen. Das Lösungsmittel wird entfernt und das erhaltene Rohprodukt säulenchromatographisch (Kieselgel, Petrolether/MTBE = 5/1 (v/v)) gereinigt. Man erhält 1.62 g (4.7 mmol, 80 %) 4-Benzyloxy-3-(1-phenyl-vinyl)-benzoesäuremethylester.

Ausbeute: 1.62 g (4.7 mmol, 80 %) 4-Benzyloxy-3-(1-phenylvinyl)-benzoesäure-methylester.
¹H-NMR (400 MHz, CDCl₃) : δ [ppm] = 3.89 (s, 3H, CH₃), 4.93 (s, 2H, OCH₂), 5.36 (d, ²J = 1.1 Hz, 1H, =CH₂), 5.70 (d, ²J = 1.1 Hz, 1H, =CH₂), 6.80 (dd, ³J = 7.6 Hz, ⁴J = 1.6 Hz, 2H, PhH), 6.95 (d, ³J = 9.0 Hz, 1H, PhH), 7.18 (m, 3H, PhH), 7.29 (m, 5H, PhH), 8.04 (m, 2H, PhH).
¹³C-NMR (100 MHz, CDCl₃): δ [ppm] = 51.9 (CH₃), 69.9 (OCH₂), 111.5 (CHₐᵣₒₘ), 116.3 (=CH₂), 122.7 (Cqₐᵣₒₘ), 126.4 (2 x CHₐᵣₒₘ), 126.7 (2 x CHₐᵣₒₘ), 127.4 (CHₐᵣₒₘ), 127.6 (CHₐᵣₒₘ), 128.2 (4 x CHₐᵣₒₘ), 131.3 (CHₐᵣₒₘ, Cqₐᵣₒₘ), 132.8 (CHₐᵣₒₘ), 136.0 (Cqₐᵣₒₘ), 141.0 (Cqₐᵣₒₘ), 146.7 (Cq_{olefin}), 159.7 (Cqₐᵣₒₘ), 166.8 (COOMe).

Schmelzpunkt: 77-78°C

### Beispiel 12: Herstellung von 4-Benzyloxy-3-(3-diisopropylamino-1-phenyl-propyl)-benzoe-säuremethylester

Ansatz:
1.50 g (4.3 mmol) 4-Benzyloxy-3-(1-phenyl-vinyl)-benzoesäuremethylester
0.45 g (4.9 mmol) Diisopropylamin
11.2 mg (0.04 mmol) Rh(acac)(CO)₂
86 µl (0.34 mmol) Tributylphosphin
15 ml abs. Dioxan

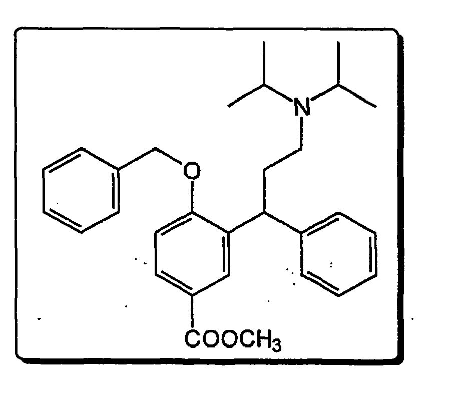

Durchführung:
analog Beispiel 5 mit p (CO/H₂) = 90/10 bar, 130°C, 65 h Die Reinigung des Rohproduktes erfolgt säulenchromatographisch (Kieselgel, Petrolether/MTBE/NEt₃ (Lösungsmittelgemisch mit steigender Polarität; PE/MTBE = 1/1 → PE/MTBE/NEt₃ = 5/5/1).

Ausbeute: 1.262 g (2.75 mmol, 63 %) 4-Benzyloxy-3-(3-diisopropylamino-1-phenyl-propyl)-benzoesäuremethylester.
¹H-NMR (400 MHz, CDCl₃): δ [ppm] = 0.89 (d, ³J = 6.5 Hz, 6H, 2 x CHC*H₃*), 0.90 (d, ³J = 6.5 Hz, 6H, 2 x CHC*H₃*), 2.17 (m, 2H, CHC*H₂*), 2.33 (m, 2H, NCH₂), 2.95 (sept, ³J = 6.5 Hz, 2H, 2 x CHMe₂), 3.87 (s, 3H, OCH₃), 4.39 (t, ³J = 7.6 Hz, 1H, CHPh₂), 5.02 (d, ²J = 15.6 Hz, 1H, OCH₂Ph), 5.05 (d, ²J = 15.6 Hz, 1H, OCH₂Ph), 6.85 (d, ³J = 8.5 Hz, 1H, PhH), 7.14 (m, 1H, PhH), 7.20 - 7.25 (m, 6H, PhH), 7.32 (m, 3H, PhH), 7.86 (dd, ³J = 8.5 Hz, ⁴J = 2.1 Hz, 1H, PhH), 8.09 (d, ⁴J = 2.1 Hz, 1H, PhH).
¹³C-NMR (100 MHz, CDCl₃): δ [ppm] = 20.5 (2 x CH*C*H₃), 20.6 (2 x CH*C*H₃), 36.6 (CH*C*H₂), 41.6 (PhCH), 43.8 (NCH₂), 48.6 (2 X NCH), 51.8 (OCH₃), 70.0 (OCH₂Ph), 111.0 (CHₐᵣₒₘ), 122.3 (Cqₐᵣₒₘ), 125.8 (CHₐᵣₒₘ), 127.4 (2 x CHₐᵣₒₘ), 127.9 (CHₐᵣₒₘ), 128.0 (2 x CHₐᵣₒₘ), 128.3 (2 x CHₐᵣₒₘ), 128.4 (2 x CHₐᵣₒₘ), 129.1 (CHₐᵣₒₘ), 129.4 (CHₐᵣₒₘ), 133.6 (Cqₐᵣₒₘ), 136.3 (Cqₐᵣₒₘ), 144.4 (Cqₐᵣₒₘ), 159.8 (Cqₐᵣₒₘ), 167.0 (COOMe).
MS (EI, 70EV): m/z [%] = 459 (M⁺, 32), 444 (49), 428 (3), 416 (1), 267 (2), 241(2), 209 (2), 165 (2), 114 (100), 91 (28), 72 (18), 57 (7).

Schmelzpunkt: 84°C

| Elementaranalyse: | (C₃₀H₃₇NO₃, M = 459.28) | | | |
|---|---|---|---|---|
| | ber.: | C 78.4 | H 8.1 | N 3.0 |
| | gef.: | C 78.4 | H 7.8 | N 2.9 |

### Beispiel 13: Hydroformylierung von 4-Methyl-2-(1-phenyl-vinyl)-phenol

*Ansatz:*
0.421 g (2.0 mmol) 4-Methyl-2-(1-phenylvinyl)-phenol
5.0 mg (0.01 mmol) [RhCl(cod)]2
10 ml abs. Dioxan

*Durchführung:*
0.421 g (2.0 mmol) 4-Methyl-2-(1-phenylvinyl)-phenol, 5.0 mg (0.01 mmol) [RhCl(cod)]₂ und 10 ml abs. Dioxan werden in einem Druckbehälter 3 Tage bei 120 °C und einem Druck von 50 bar Synthesegas (CO:H₂ = 3:2) gerührt. Nach Abkühlen und Entspannen des Druckbehälters wird die Reaktionslösung über Aluminiumoxid (Aktivität II - III, basisch) absorptiv filtriert (Elutionsmittel: Diethylether). Nach Entfernen des Lösungsmittels erfolgt die Reinigung des Rohproduktes säulenchromatographisch an Kieselgel mit Toluol/MTBE (Volumenverhältnis = 3/1) als Elutionsmittel. Man erhält 310 mg (1.3 mmol, 64 %) 4-Methyl-2-(1-phenylvinyl)-phenol als Gemisch zweier Diastereomere im Verhältnis 4:1.

*Ausbeute:* 310 mg (1.3 mmol, 64 %) 6-Methyl-4-phenyl-chroman-2-ol.

*Spektroskopische Daten:* 6-Methyl-4-phenyl-chroman-2-ol. 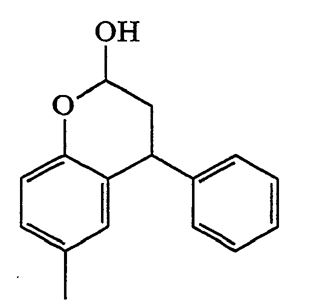 *¹H-NMR (400 MHz, CDCl₃):* δ [ppm] = 2.06 (m, 4 H, CH₃ + CHH), 2.17 (ddd, ³J = 13.6 Hz, ³J = 5.8 Hz, ²J = 3.8 Hz, 1 H, CHH), 4.21 (dd, ³J = 10. 8 Hz, ³J = 5.8 Hz, 1 H, CHArAr'), 5.41/5.54 (m, 1 H, C*H*OH), 6.50 (s, 1 H, PhH), 6.70 (m, 1 H, PhH), 6.85 (m, 1 H, PhH), 7.10-7.26 (m, 5 H, PhH).
*¹³C-NMR (100 MHz, CDCl₃): δ* [ppm] = 20.5 (CH₃), 36.3 (CH₂), 36.9 (CHArAr'), 91.2/90.7 (CHOH), 116.5/116.6 (CHₐᵣₒₘ), 124.8/124.7 (Cqₐᵣₒₘ), 126.6/126.8 (CHₐᵣₒₘ), 128.5/128.7 (2 x CHₐᵣₒₘ), 128.5/128.4 (CHₐᵣₒₘ), 128.8/128.4 (2 x CHₐᵣₒₘ), 129.8/129.5 (CHₐᵣₒₘ), 130.1/130.2 (Cqₐᵣₒₘ), 144.4/144.0 (Cqₐᵣₒₘ), 149.6/151.1 (Cqₐᵣₒₘ).

Das erhaltene Lactol läßt sich nach an sich bekannten Verfahren zu den gewünschten Diarylpropylaminen durch reduktive Aminierung umsetzen.

### Beispiel 14: Hydroaminomethylierung von 1-Benzyloxy-4-methyl-2-(1-phenylvinyl)-benzol mit chiralen Liganden

*Ansatz:*
0.250 g (0.83 mmol) 1-Benzyloxy-4-methyl-2-(1-phenylvinyl)-benzol
0.095 g (0.94 mmol) Diisopropylamin
4.3 mg (0.02 mmol) Rh(acac)(CO)₂
s. Tabelle 4 Ligand
10 ml abs. Dioxan

*Durchführung:*
0.250 g (0.83 mmol) 1-Benzyloxy-4-methyl-2-(1-phenylvinyl)-benzol, 0.095 g (0.94 mmol) Diisopropylamin, 4.3 mg (0.02 mmol) Rh(acac)(CO)₂, eine definierte Menge Ligand (s. Tabelle 4) und 10 ml abs. Dioxan werden in einem Druckbehälter unter den in Tabelle 4 angegebenen Bedingungen gerührt. Nach Abkühlen und Entspannen des Druckbehälters wird die Reaktionslösung über Aluminiumoxid (Aktivität II - III, basisch) absorptiv filtriert (Elutionsmittel: MTBE). Nach Entfernen des Lösungsmittels erfolgt die Reinigung des Rohproduktes säulenchromatographisch an Aluminiumoxid (Aktivität II-III, neutral) mit Cyclohexan/MTBE (Volumenverhältnis = 5/1) als Elutionsmittel.

**Tabelle 4: Hydroaminomethylierung mit 1-Benzyloxy-4-methyl-2-(1-phenylvinyl)-benzol unter Verwendung chiraler Diphosphinliganden**

| Nr. | Ligand | L/Rh | CO/H₂ [bar | T [ba | t [d | Pro- [%] ^{*} | [α]_{D}²⁰ [°] | ee^{**} [%] |
|---|---|---|---|---|---|---|---|---|
| | | | 0 | | | | | |
| 3 | (R)-BINAP | 4/1 | 90/1 | 130 | 3 | 57 | + | < 2 |
| | | | 0 | | | | 0.54 | |
| 4 | (R)-BINAP | 4/1 | 90/1 | 130 | 1 | 58 | + | < 2 |
| | | | 0 | | | | 0.53 | |
| 5 | (R)-BINAP | 2/1 | 70/3 | 115 | 3 | 60 | + | < 2 |
| | | | 0 | | | | 0.26 | |
| 6 | (R)-BINAP | 2/1 | 60/2 | 105 | 3 | 45 | + | < 2 |
| | | | 0 | | | | 0.28 | |

| | | | | | | | | |
|---|---|---|---|---|---|---|---|---|
| ^{*}: GC-Anteile ^{**}: hydriertes Edukt jeweils nur untergeordnet zu detektieren (< 5 %) | | | | | | | | |

### Beispiel 15: Herstellung von 4-[4-Methyl-2-(1-phenyl-vinyl)-phenoxy]-3,5-dioxa-4-phospha-cyclohepta[2,1-a-3,4-a']dinaphtalin

*Ansatz:*
0.50 g (2.4 mmol) 1-Hydroxy-4-methyl-2-(1-phenylvinyl)-benzol
1.05 g (3.0 mmol) *(S)*-(1,1-Binaphtalen-2,2'-dioxy)-chloro-phosphin
0.33 g (3.3 mmol) Triethylamin
55 ml Diethylether

*Durchführung:*
Zu einer Lösung aus 0.50 g (2.4 mmol) 1-Hydroxy-4-methyl-2-(1-phenylvinyl)-benzol und 1.05 g (3.0 mmol) *(S)*-(1,1-Binaphthalen-2,2'-dioxy)chloro-phosphin in 45 ml Diethylether werden bei 0 °C 0.33 g (3.3 mmol) Triethylamin in 10 ml Diethylether zugegeben. Die so erhaltene Suspension rührt man 18 h bei RT und quencht anschliessend mit 50 ml Brine. Die organische Phase wird abgetrennt und die wässrige Phase zweimal mit je 50 ml Diethylether extrahiert. Die vereinigten organischen Phasen trocknet man über MgSO₄ und entfernt das Lösungsmittel anschliessend am Rotationsverdampfer. Die Reinigung erfolgt säulenchromatographisch an Kieselgel mit Cyclohexan/Ethylacetat (Verhältnis 2/1) als Elutionsmittel. Man erhält 0.73 g (1.4 mmol, 59 %) 4-[4-Methyl-2-(1-phenyl-vinyl)-phenoxy]-3,5-dioxa-4-phospha-cyclohepta[2,1-*a*-3,4-*a*']dinaphthalin als weissen Feststoff.

*Ausbeute:* 0.73 g (1.4 mmol, 59 %) 4-[4-Methyl-2-(1-phenyl-vinyl)-phenoxy]-3,5-dioxa-4-phospha-cyclohepta[2,1-*a*-3,4-*a*']dinaphthalin.

*Spektroskopische Daten:* 4-[4-Methyl-2-(1-phenyl-vinyl)-phenoxy]-3,5-dioxa-4-phospha-cyclohepta[2,1-*a*-3,4-*a'*]dinaphtalen 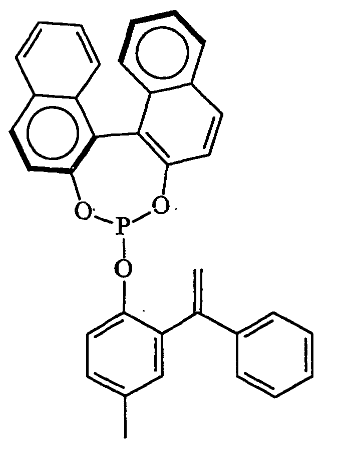 *¹H-NMR (400 MHz, CDCl₃): δ* [ppm] = 2.33 (s, 3 H, CH₃), 5.35 (d, ²J = 1.0 Hz, 1 H =CH₂), 5.79 (d, ²J = 1.0 Hz, 1 H, =CH₂), 7.02 (d, ³J = 8.8 Hz, 1 H, PhH), 7.13 (m, 3 H, PhH), 7.20 (m, 2 H, PhH), 7.28-7.38 (m, 10 H, PhH), 7.75 (d, ³J = 8.8 Hz, 1 H, PhH), 7.87 (m, 3 H, PhH).
*¹³C-NMR (100 MHz, CDCl₃):* δ [ppm] = 20.7 (CH₃), 116. 9 (=CH₂), 121.1 (2 x CHₐᵣₒₘ), 121.8 (CHₐᵣₒₘ), 124.8 (CHₐᵣₒₘ), 126.1 (2 x CHₐᵣₒₘ), 126.8 (2 x CHₐᵣₒₘ), 126.9 ( 2 x CHₐᵣₒₘ), 127.7 (CHₐᵣₒₘ), 128.2 (2 x CHₐᵣₒₘ), 128.4 (2 x CHₐᵣₒₘ), 129.6 (2 x CHₐᵣₒₘ), 130.2 (CHₐᵣₒₘ), 131.3 (2 x Cqₐᵣₒₘ), 132.2 (2 x CHₐᵣₒₘ), 132.6 (2 x Cqₐᵣₒₘ), 134.1 (Cqₐᵣₒₘ), 134.2 (Cqₐᵣₒₘ), 140.5 (Cqₐᵣₒₘ), 146.1 (2 x Cqₐᵣₒₘ), 146.7 (Cqₐᵣₒₘ, ²J_{P-C} = 7.8 Hz), 147.0 (Cq_{olefin}), 147.7 (2 x Cqₐᵣₒₘ, ²J_{P-C} = 4.9 Hz).
*³¹P-NMR (202 MHz, CDCl₃):* δ [ppm] = 158. 6
*MS (EI, 70EV) :* m/z [%] = 524 (M⁺, 100), 447 (13), 315 (13), 268 (59), 239 (42), 209 (7), 105 (5), 77 (2).

### Schmelzpunkt: 95 °C

### Beispiel 16: Herstellung von 2-[4-Methyl-2-(1-phenyl-vinyl)-phenoxymethyl]-pyrrolidin-1-carbonsäure-tert-butylester

*Ansatz:*
2.09 g (9.9 mmol) 1-Hydroxy-4-methyl-2-(1-phenylvinyl)-benzol
2.00 g (9.9 mmol) N-Boc-*(S)*-prolinol
1.90 g (10.9 mmol) Diethylazodicarboxylat
2.86 g (10.9 mmol) Triphenylphosphin
50 ml abs. THF

*Durchführung:*
Zu einer Lösung aus 2.09 g (9.9 mmol) 1-Hydroxy-4-methyl-2-(1-phenylvinyl)-benzol, 2.00 g (9.9 mmol) N-Boc-(*S*)-prolinol und 2.86 g (10.9 mmol) Triphenylphosphin in 50 ml THF werden 1.90 g (10.9 mmol) Diethylazodicarboxylat getropft. Das Reaktionsgemisch wird 20 h unter Rückfluss erhitzt. Anschliessend entfernt man das Lösungsmitel am Rotationsverdampfer, nimmt den Rückstand in Dichlormethan auf und filtriert die Lösung durch Aluminiumoxid (basisch, Aktivität II - III). Nach Entfernen des Lösungsmittels wird das so erhaltene Rohprodukt säulenchromatographisch an Kieselgel mit Toluol/MTBE (Volumenverhältnis = 20/1) als Elutionsmittel gereinigt. Man erhält 3.13 g (7.9 mmol, 80 %) 2-[4-Methyl-2-(1-phenyl-vinyl)-phenoxymethyl]-pyrrolidin-1-carbonsäure-*tert*-butylester.

*Ausbeute:* 3.13 g (7.9 mmol, 80 %) 2-[4-Methyl-2-(1-phenyl-vinyl)-phenoxymethyl]-pyrrolidin-1-carbonsäure-*tert-*butylester

*Spektroskopische Daten:* 2-[4-Methyl-2-(1-phenyl-vinyl)-phenoxymethyl]-pyrrolidin-1-carbonsäure-*tert*-butylester (1.5/1 Diastereomerengemisch) 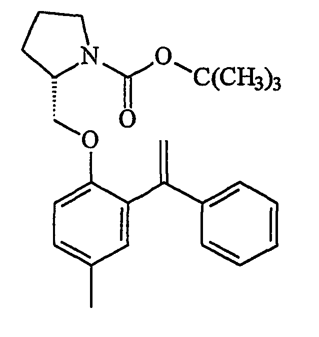 *¹H-NMR (400 MHz, CDCl₃):* δ [ppm] = 1.26 (m, 1.4 H NCH₂NC*H*₂), 1.43 (s, 9H, C(C*H*₃)₃), 1.54 (m, 2.6 H, NCH₂C*H*₂, NCHC*H*₂), 2.30 und 2.31 (2 x s, 3 H, CH₃), 3.02 - 3.25 (m, 2 H, NCH₂), 3.54 (*t, ³J = 8.5 Hz, 0.6 H, OCH₂), 3.70 - 3.94 (m, 2.4 H, OCH₂, NCH), 5.27 (d, ²J = 4.3 Hz, 1H, =CH₂), 5.66 (s, 1 H, =CH₂), 6.87 (m, 1 H, PhH), 6.96 - 7.29 (m, 7 H, PhH).
*¹³C-NMR (100 MHz, CDCl₃): δ* [ppm] = 20.4 (CH₃), 22.6 und 23.7 (CH₂), 27.3 und 28.0 (CH₂), 28.5 (3 x C(CH₃)₃), 46.4 und 46.8 (NCH₂), 55.7 und 55.9 (NCH), 68.0 und 68.3 (OCH₂), 79.0 und 79.4 (CMe₃), 111.9 (CHₐᵣₒₘ), 114.9 und 115.2 (=CH₂), 125.7 und 126.0 (CHₐᵣₒₘ), 126.3 (2 x CHₐᵣₒₘ), 128.0 (2 x CHₐᵣₒₘ), 128.2 und 128.9 (CHₐᵣₒₘ), 130.0 und 130.7 (Cqₐᵣₒₘ), 131.8 und 132.0 (CHₐᵣₒₘ), 137.7 (Cqₐᵣₒₘ), 141.4 (Cqₐᵣₒₘ), 147.5 (Cq_{olefin}), 154.0 (Cqₐᵣₒₘ), 154.4 und 154.5 (NCOO^{t}Bu).
*MS (EI, 70EV) :* m/z [%] = 393 (M⁺, 15) , 337 (4) , 320 (5), 293 (33), 278 (2), 226 (4), 209 (44), 195 (16), 165 (19), 128 (42), 114 (62), 70 (87), 57 (100).

*spezifische Drehung:*
[α]_{D}²⁰ = - 45.5 ° (c = 0.89, Diethylether)

| *Elementaranalyse:* | (C₂₅H₃₁NO₃, M = 393.23) | | | |
|---|---|---|---|---|
| | ber.: | C 76.3 | H 7.9 | N 3.6 |
| | gef.: | C 76.0 | H 8.0 | N 3.5 |

### Beispiel 17: Herstellung von 2-[4-Methyl-2-(1-phenyl-vinyl)-phenoxymethyl]-pyrrolidin

*Ansatz:*
1.76 g (4.5 mmol) 2-[4-Methyl-2-(1-phenyl-vinyl)-phenoxymethyl]-pyrrolidin-1-carbonsäure-tert-butylester
5.4 ml Trifluoressigsäure
22 ml abs. CH₂Cl₂

*Durchführung:*
Zu einer Lösung aus 1.76 g (4.5 mmol) 2-[4-Methyl-2-(1-phenyl-vinyl)-phenoxymethyl]-pyrrolidin-1-carbonsäure-tert-butylester in 22 ml abs. CH₂Cl₂ werden bei 0 °C 5.4 ml Trifluoressigsäure gegeben und man rührt die Reaktionslösung 1 h bei derselben Temperatur. Anschließend engt man das Reaktionsgemisch am Rotationsverdampfer ein und nimmt den Rückstand in 20 ml Dichlormethan auf. Die Lösung wird mit 20 ml konzentrierter Ammoniaklösung versetzt und man trennt die organische Phase ab. Die wässrige Phase wird zweimal mit je 10 ml Dichlormethan extrahiert und die vereinigten organischen Phasen werden über MgSO₄ getrocknet. Das Lösungsmittel entfernt man am Rotationsverdampfer und das so erhaltene Rohprodukt wird säulenchromatographisch an Kieselgel mit MTBE/Cyclohexan/Ethanol/Diisopropylamin (Volumenverhältnis = 3/1/1 + Vol.-%) als Elutionsmittel gereinigt. Man erhält 1.31 g (4.5 mmol, 100 %) 2-[4-Methyl-2-(1-phenyl-vinyl)-phenoxymethyl]-pyrrolidin.

*Ausbeute:* 1.31 g (4.5 mmol, 100 %) 2-[4-Methyl-2-(1-phenyl-vinyl)-phenoxymethyl]-pyrrolidin.

*Spektroskopische Daten:* 2-[4-Methyl-2-(1-phenyl-vinyl)-phenoxymethyl]-pyrrolidin. 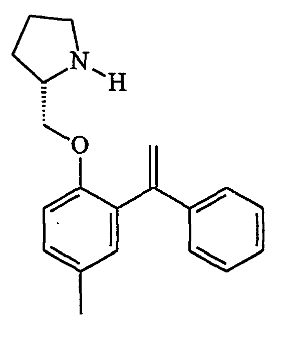 *¹H-NMR (400 MHz, CDCl₃):* δ [ppm] = 1.18 (m, 1 H NCH₂NC*H*₂), 1. 5 6 (m, 3 H, NCH₂C*H*₂, NCHC*H*₂), 1.85 (s (b), 1 H, NH), 2.33 (s, 3 H, CH₃), 2.57 (m, 1 H, NCH₂), 2.65 (m, 1 H, NCH₂), 3.00 (m, 1 H, NCH), 3.64 (dd, ²J = 8.9 Hz, ³J = 6.3 Hz, 1 H, OCH₂), 3.77 (dd, ²J = 8.9 Hz, ³J = 4.0 Hz, 1 H, OCH₂), 5.30 (d, ²J = 1.0 Hz, 1H, =CH₂), 5.60 (d, ²J = 1.0 Hz, 1 H, =CH₂), 6.73 (d, ³J = 8.3 Hz, 1 H, PhH), 7.08 - 7.14 (m, 2 H, PhH), 7.24 - 7.30 (m, 5 H, PhH).
*¹³C-NMR (100 MHz, CDCl₃):* δ [ppm] = 20.4 (CH₃), 24.8 (CH₂), 26.9 (CH₂), 46.2 (NCH₂), 57.4 (NCH), 69.7 (OCH₂), 111.4 (CHₐᵣₒₘ), 115.4 (=CH₂), 126.2 (2 x CHₐᵣₒₘ), 127.2 (CHₐᵣₒₘ), 128.1 (2 X CHₐᵣₒₘ), 129.4 (CHₐᵣₒₘ), 129.8 (Cqₐᵣₒₘ), 130.6 (Cqₐᵣₒₘ), 131.9 (CHₐᵣₒₘ), 141.8 (Cqₐᵣₒₘ), 147.9 (Cq_{olefin}), 153.8 (Cqₐᵣₒₘ).
*MS (EI, 70EV) :* m/z [%] = 293 (M⁺, 15), 211 (4), 209 (4), 195 (1), 178 (3), 165 (6), 152 (2), 70 (100).

*spezifische Drehung:*
[α]_{D}²⁰ = + 15.3 ° (c = 1.00, Diethylether)

| *Elementaranalyse:* | (C₂₀H₂₃NO, M = 239.2) | | | |
|---|---|---|---|---|
| | ber.: | C 81.9 | H 7.9 | N 4.8 |
| | gef.: | C 81.3 | H 7.9 | N 4.5 |

### Beispiel 18: Herstellung von Diphenylphosphanyl-[4-methyl-2-(1-phenyl-vinyl)-phenoxymethyl]-pyrrolidin

*Ansatz:*
1.31 g (4.5 mmol) 2-[4-Methyl-2-(1-phenyl-vinyl)-phenoxymethyl]-pyrrolidin
1.00 g (4.6 mmol) P-Chlordiphenylphosphin
1.13 g (11.2 mmol) Triethylamin
15 ml abs. Toluol

*Durchführung:*
Zu einer Lösung aus 1.31 g (4.5 mmol) 2-[4-Methyl-2-(1-phenyl-vinyl)-phenoxymethyl]-pyrrolidin und 1.13 g (11.2 mmol) Triethylamin in 12 ml abs. Toluol werden bei 0 °C innerhalb 30 min 1.00 g (4.6 mml) P-Chlordiphenylphosphin in 3 ml abs. Toluol getropft. Die Reaktionslösung lässt man 20 h bei RT rühren. Anschliessend wird von entstandenem Triethylammoniumchlorid abfiltriert, der Rückstand mit 20 ml Toluol nachgewaschen und die Filtrate am Rotionsverdampfer eingeengt. Die Reinigung des erhaltenen Rohproduktes erfolgt säulenchromatographisch an Kieselgel mit MTBE/Cyclohexan/Diisopropylamin (Volumenverhältnis = 5/1 + 10 Vol-%) als Elutiosmittel. Man erhält 1.31 g (2.7 mmol, 61 %) Diphenylphosphanyl-[4-methyl-2-(1-phenyl-vinyl)-phenoxymethyl]-pyrrolidin.

*Ausbeute:* 1.31 g (2.7 mmol, 61 %) Diphenylphosphanyl-[4-methyl-2-(1-phenyl-vinyl)-phenoxymethyl]-pyrrolidin.

*Spektroskopische Daten:* Diphenylphosphanyl-[4-methyl-2-(1-phenyl-vinyl)-phenoxymethyl]-pyrrolidin 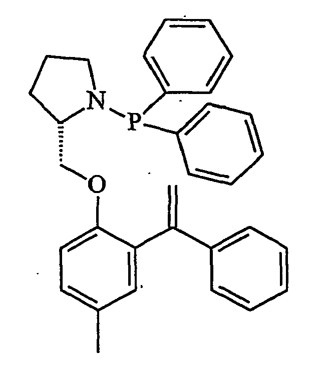 *¹H-NMR (400 MHz, CDCl₃):* δ [ppm] = 1.26 (m, 1 H NCH₂NC*H*₂), 1.44 (m, 3 H, NCH₂C*H*₂, NCHC*H*₂), 2.31 (s, 3 H, CH₃), 2.53 (m, 1 H, NCH₂), 2.80 (m, 1 H, NCH₂), 3.48 (m, 1 H, NCH), 3.62 (m, 1 H, OCH₂), 3. 92 (m, 1 H, OCH₂), 5.29 (d, ²J = 1.5 Hz, 1H, =CH₂), 5.64 (d, ²J = 1.5 Hz, 1 H, =CH₂), 6.78 (d, ³J = 8.5 Hz, 1 H, PhH), 7.07 (m, 2 H, PhH), 7.19 - 7.34 (m, 15 H, PhH).
*¹³C-NMR (100 MHz, CDCl₃):* δ [ppm] = 20.4 (CH₃), 25.1 (NCH₂CH₂), 28.9 (NCHCH₂, ³J_{C-P} = 5.8Hz), 47.0 (NCH₂, ²J_{C-P} = 8.7 Hz), 61.4 (NCH, ²J_{C-P} = 29.2 Hz), 70.8 (OCH₂, ³J_{C-P} = 5.8 Hz), 112.0 (CHₐᵣₒₘ), 115.1 (=CH₂), 126.4 (3 x CHₐᵣₒₘ), 127.1 (CHₐᵣₒₘ), 127.8 (CHₐᵣₒₘ), 127.9 (3 x CHₐᵣₒₘ), 128.0 (2 x CHₐᵣₒₘ), 128.9 (2 x CHₐᵣₒₘ), 129.6 (Cqₐᵣₒₘ), 130.8 (Cqₐᵣₒₘ), 131.2 (2 x CHₐᵣₒₘ), 131.9 (CHₐᵣₒₘ), 132.7 (2 x CHₐᵣₒₘ), 138.5 (P-Cqₐᵣₒₘ, ¹J_{P-C} = 16.5 Hz), 139.5 (P-Cqₐᵣₒₘ, ¹J = 6.8 Hz), 141.6 (Cqₐᵣₒₘ), 147.7 (Cq_{olefin}), 154.1 (Cqₐᵣₒₘ). *³¹P-NMR (202 MHz, CDCl₃):* δ [ppm] = 46.2.
*MS (EI, 70EV):* m/z [%] = 477 (M⁺, 15), 395 (37), 379 (15), 317 (1), 292 (6), 280 (5), 267 (11), 254 (54), 185 (100), 152 (10), 91 (9).

### Beispiel 19: Hydroaminomethylierung von 4-Methyl-2-(1-phenyl-vinyl)-phenol

*Ansatz:*
0.210 g (1.0 mmol) 4-Methyl-2-(1-phenylvinyl)-phenol
0.10 ml (1.15 mmol) Morpholin
6.0 mg (12 µmol) [RhCl(cod]]₂
10 ml abs. Dioxan

*Durchführung:*
0.421 g (2.0 mmol) 4-Methyl-2-(1-phenylvinyl)-phenol, 0.10 ml (1.15 mmol) Morpholin, 6.0 mg (12 µmol) [RhCl(cod)]₂ und 10 ml abs. Dioxan werden in einem Druckbehälter 3 Tage bei 120 °C und einem Druck von 90 bar Synthesegas (CO:H₂ = 7:2) gerührt. Nach Abkühlen und Entspannen des Druckbehälters wird die Reaktionslösung über Aluminiumoxid (Aktivität II - III, basisch) absorptiv filtriert (Elutionsmittel: Diethylether, anschließend Ethanol). Nach Entfernen der Lösemittels erfolgt die Reinigung des Rohproduktes säulenchromatographisch an Kieselgel mit Cyclohexan/MTBE (Volumenverhältnis = 1/1) als Elutionsmittel. Man erhält 255 mg (0.82 mmol, 82 %) 4-Methyl-2-(3-morpholin-4-yl-1-phenyl-propyl)-phenol.

*Ausbeute:* 255 mg (0.82 mmol, 82 %) 4-Methyl-2-(3-morpholin-4-yl-1-phenyl-propyl)-phenol.

*Spektroskopische Daten:*4-Methyl-2-(3-morpholin-4-yl-1-phenylpropyl)-phenol. 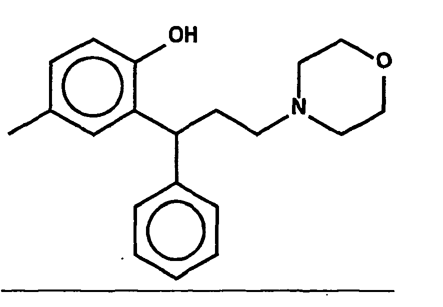 *¹H-NMR (400 MHz, CDCl₃):* δ [ppm] = 2.09 (s, 3 H, CH₃), 2.31-2.49 (6 H, CH₂ + 2x NCH₂), 2.65 (brs, 2 H, NCH₂), 3.78-3.89 (4 H, 2x OCH₂), 4.44 (dd, ³*J* = 12.8, 3.7 Hz, CHArAr'), 6.48 (s, 1 H, OH), 6.85 (m, 2 H, Ar-H), 7.19-3.33 (6 H, Ar-H).
*¹³C-NMR (100 MHz, CDCl₃):* δ [ppm] = 20.6 (CH₃), 30.4 (CH₂), 38.1 (CHArAr'), 54.5 (2x NCH₂), 58.3 (NCH₂), 66.3 (2x OCH₂), 117.4 (CHₐᵣₒₘ), 126.2 (CHₐᵣₒₘ), 128.0 (2x CHₐᵣₒₘ), 128.3 (2x CHₐᵣₒₘ), 129.2 (2x CHₐᵣₒₘ), 129.5 (Cqₐᵣₒₘ), 130.7 (Cqₐᵣₒₘ), 144.5 (Cqₐᵣₒₘ), 153.4 (Cqₐᵣₒₘ).

### Synthese von rac-Tolterodin

*Ansatz:*
0.210 g (1.0 mmol) 4-Methyl-2-(1-phenylvinyl)-phenol
0.17 ml (1.2 mmol) Diisopropylamin
6.0 mg (12 µmol) [RhCl(cod]]₂
10 ml abs. Dioxan

*Durchführung:*
0.210 g (1.0 mmol) 4-Methyl-2-(1-phenylvinyl)-phenol, 0.17 ml (1.2 mmol) Diisopropylamin, 6.0 mg (12 µmol) [RhCl(cod)]₂ und 10 ml abs. Dioxan werden in einem Druckbehälter 3 Tage bei 120 °C und einem Druck von 90 bar Synthesegas (CO:H₂ = 7:2) gerührt. Nach Abkühlen und Entspannen des Druckbehälters wird die Reaktionslösung über Aluminiumoxid (Aktivität II - III, basisch) absorptiv filtriert (Elutionsmittel: Diethylether, anschließend Ethanol). Die Lösemittel werden abdestilliert. Man erhält 295 mg eines Rohprodukts, das lt. NMR zu 71 % 2-(3-Diisopropylamino-1-phenyl-propyl)-4-methyl-phenol und zu 29 % 6-Methyl-4-phenyl-chroman-2-ol enthält.

*Ausbeute:* 228 mg (0.70 mmol, 70 %) 2-(3-Diisopropylamino-1-phenyl-propyl)-4-methyl-phenol
*Spektroskopische Daten:*
2-(3-Diisopropylamino-1-phenyl-propyl)-4-methyl-phenol

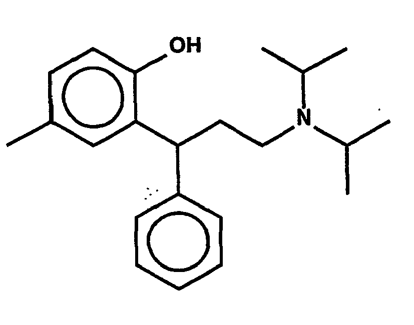

Die spektroskopischen Daten entsprechen denen der Literatur P.G. Andersson, H. E. Schenk, K. Österlund *J. Org. Chem.* **1998**, 63, 8067.

## Patentansprüche

1. Verfahren zur Herstellung von 3,3-Diarylpropylaminen der allgemeinen Formel (I) 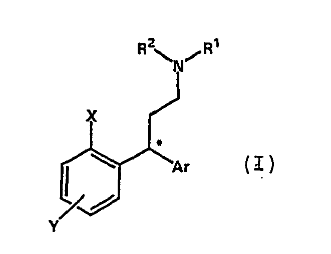 worin
Ar für einen substituierten oder unsubstituierten Arylrest steht,
X für OH oder OR³ steht,
Y für Cl, Br, I, CN, CHO, CH₂OR, COOH, COOR, worin R für C₁-C₁₀-Alkyl oder einen substituierten oder unsubstituierten Arylrest steht, oder C₁-C₁₀-Alkyl steht,
R¹, R² für C₁-C₁₀-Alkyl oder C₃-C₈-Cycloalkyl steht, wobei R¹ und R² unter Bildung einer cyclischen Struktur verknüpft sein können,
und wobei R³ für einen Rest steht, der von einer der folgenden Verbindungen abgeleitet ist:
(i) den Aminosäuren D-Prolin, L-Prolin, D-Alanin, L-Alanin, D-Asparagin, L-Asparagin, D-Asparaginsäure, L-Asparaginsäure, D-Glutamin, L-Glutamin, D-Glutaminsäure, L-Glutaminsäure, D-Phenylalanin, L-Phenylalanin, D-Histidin, L-Histidin, D-Leucin, L-Leucin, D-Serin, L-Serin, L-Threonin, D-Threonin, D-Tryptophan, L-Tryptophan, D-Tyrosin, L-Tyrosin, D-Valin, L-Valin, D-Cystein, L-Cystein, D-Methionin, L-Methionin, D-Isoleucin, L-Isoleucin, oder den Alkoholen, die sich aus diesen Aminosäuren durch Reduktion der Carbonsäurefunktion zur Hydroxymethyleneinheit ergeben,
(ii) den Aminosäuren N-Diphenylphosphanyl-D-alanin, N-Diphenylphosphanyl-L-alanin, N-Diphenylphosphanyl-D-prolin, N-Diphenylphosphanyl-L-prolin, N-Diphenylphosphanyl-D-phenylalanin, N-Diphenylphosphanyl-L-phenylalanin, sowie den Alkoholen, die sich aus diesen Aminosäuren durch Reduktion der Carbonsäurefunktion zur Hydroxymethyleneinheit ergeben,
(iii) α-Hydroxycarbonsäurederivaten der allgemeinen Formel 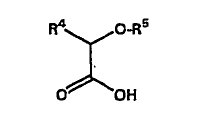 jeweils in Form beider optischen Antipoden, worin R⁴ für eine lineare oder verzweigte C₁-C₁₀-Alkylgruppe oder Cycloalkylgruppe oder einen substituierten oder unsubstituierten Arylrest und R⁵ für C₁-C₁₀-Alkyl, Cycloalkyl, Acyl, Alkoxycarbonyl, Benzoyl, Diphenylphosphanyl, Dicyclohexylphosphanyl oder Diarylphosphanyl steht,
(iv) den Verbindungen der allgemeinen Formeln 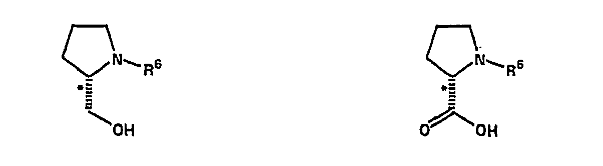 worin R⁶ für einen Substituenten ausgewählt aus der Gruppe bestehend aus PPh₂, P(C₆H₁₁)₂, P(Aryl)₂, Alkyl, Acyl, Alkoxycarbonyl, Benzoyl, Arylcarbonyl, Diarylphosphanyl und Dicyclohexylphosphanyl steht, und deren Stereoisomere,
(v) den Verbindungen der allgemeinen Formeln 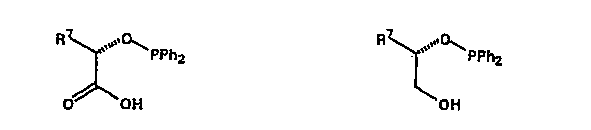 worin R⁷ für eine lineare oder verzweigte C₁-C₁₀-Alkylgruppe oder einen substituierten oder unsubstituierten Arylrest steht, und'Stereoisomere davon,
(vi) den Säuren
(R)-Acetoxyphenylessigsäure,
(R)- und (S)-1,4-Benzodioxan-2-carbonsäure,
(R)-(-)- und (S)-(+)-Hexahydro-acetoxymandelsäure,
(2R,3S)-2,3-O-Isopropyliden-2,3-dihydroxybuttersäure und deren Stereoisomere,
(+)- und (-)-Menthyloxyessigsäure,
(R)- und (S)-3-Phenyl-2-acyloxypropionsäure,
(R)- und (S)-Acetoxymandelsäure,
(R)- und (S)-α-Methoxy-α-trifluormethylphenylessigsäure,
(S)-(+)-alpha-Methoxyphenylessigsäure,
(R)- und (S)-5-Oxo-tetrahydrofuran-2-carbonsäure, sowie den Alkoholen, die sich aus diesen Säuren durch Reduktion der Carbonsäurefunktion zur Hydroxymethyleneinheit ergeben,
(vii) Verbindungen der allgemeinen Formel 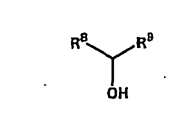 worin R⁸ für einen substituierten oder unsubstituierten Arylrest und R⁹ für Wasserstoff oder für einen linearen oder verzweigten C₁-C₁₀-Alkylrest steht,
(viii) α-Naphthol, β-Naphthol oder (R)- oder (S)-1-(9-Anthryl)-2,2,2-trifluoroethanol,
(ix) 2-Methylamino-1-phenyl-propan-1-ol (Ephedrin) aller stereomerer Formen,
oder R³ für einen der folgenden Reste steht:
(x) Phosphitreste der allgemeinen Formel -P(OR¹⁰) (OR¹¹), worin R¹⁰ und R¹¹ gleich oder verschieden sein können und für einen gegebenenfalls polycyclischen oder verbrückten Arylrest stehen,
(xi) C₁-C₁₀-Alkyl, verzweigt oder linear,
(xii) Acyl,
(xiii) Benzyl oder substituierte Benzylreste,
**dadurch gekennzeichnet, dass** man Verbindungen der allgemeiner Formel (II) 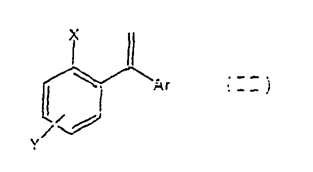 worin X, Y und Ar wie voranstehend definiert sind, mit Kohlenmonoxid (CO) und Wasserstoff (H₂) in Gegenwart eines Übergangsmetallkatalysators miteinander umsetzt und die resultierenden Oxoverbindungen in Gegenwart eines Übergangsmetallkatalysators in einer Eintopfreaktion direkt mit einem Amin der allgemeinen Formel HNR¹R², worin R¹ und R² wie oben definiert sind, umsetzt, wobei der verwendete Katalysator ein oder mehrere Übergangsmetalle, ausgewählt aus der Gruppe bestehend aus Ruthenium, Rhodium, Platin, Kobalt, Iridium, palladium und Nickel umfasst, und in situ aus einem Katalysatorvorläufer und einem Liganden, ausgewählt aus der Gruppe bestehend aus
(2-(Diphenylphosphino)-1,1'-binaphthalen-2'-yl)-1,2'-binaphthalen-2,2'-diyl-phosphit (BINAPHOS),
(2,2-Dimethyl-4,5-diphenylphosphinomethyl)-1,3-dioxolan (DIOP),
(2,2-Dimethyl-4,5-bis(5H-dibenzophosphol-5-ylmethyl)-1,3-dioxolan,
1,4-Bis(diphenylphosphino)butan,
2,3-Bis(diphenylphosphino)butan,
1,2-Bis(diphenylphosphinomethyl)cyclobutan,
1,2-Bis(5H-dibenzophosphcl-5-ylmethyl)cyclobutan,
1,2-Bis(diphenylphosphinomethyl)cyclohexan,
1,2-Bis(5H-dibenzophosphol-5-ylmethyl)cyclohexan,
(2S,4S)-N-tert.-Butoxycarbonyl-4-diphenylphosphino-2-diphenylphosphinomethyl-pyrrolicin (BPPM),
1,2-Bis(diphenylphosphinoxy)cyclohexan,
Benzyl-methyl-phenylphosphin,
Cyclohexyl-o-anisyl-methylphosphin,
Neomenthyldiphenylphosphin,
Phenyl-o-anisyl-methylphosphin,
ortho-Diphenylphosphanylbenzoyl
Tributylphosphin,
2,2'-Bis(diphenylphosphino)-1,1'-binaphthyl (BINAP) und Gemischen bzw. Stereoisomeren davon, gebildet wird.

2. Verfahren nach Anspruch 1, **dadurch gekennzeichnet, dass** man Verbindungen der allgemeinen Formel (IV) 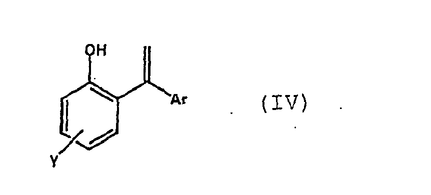 worin Y und Ar wie in Anspruch 1 definiert sind, mit Kohlenmonoxid (CO) und Wasserstoff (H₂) zu einem Chromen/Lactol-System 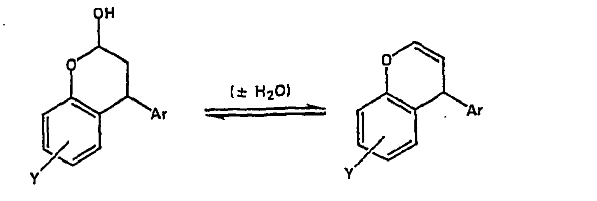 worin Y und Ar wie in Anspruch 1 definiert sind, umsetzt, und das Umsetzungsprodukt mit einem Amin der allgemeinen Formel HNR¹R², worin R¹ und R² wie in Anspruch 1 definiert sind, umsetzt.

3. Verfahren nach Anspruch 1 oder 2, **dadurch gekennzeichnet, dass** man es bei einer Temperatur von 50 bis 200°C, vorzugsweise 100 bis 140°C, durchführt.

4. Verfahren nach den Ansprüchen 1 bis 3, **dadurch gekennzeichnet, dass** man es bei einem Druck von 40 bis 200 bar, vorzugsweise 80 bis 120 bar, durchführt.

5. Verfahren nach Anspruch 4, **dadurch gekennzeichnet, dass** das Druckverhältnis CO/H₂ 10/90 bis 90/10, vorzugsweise 70/30 bis 90/10, beträgt.

6. Verfahren nach den Ansprüchen 1 bis 5, **dadurch gekennzeichnet, dass** man die Reaktion über einen Zeitraum von 2 Stunden bis 4 Tagen, vorzugsweise 1 bis 3 Tagen, durchführt.

7. Verfahren nach den Ansprüchen 1 bis 6, **dadurch gekennzeichnet, dass** der Katalysator Rhodium umfasst.

8. Verfahren nach den Ansprüchen 1 bis 7, **dadurch gekennzeichnet, dass** der Katalysatorvorläufer [Rh(cod)Cl]₂ und/oder Rh(acac)(CO)₂ ist.

9. Verfahren nach den Ansprüchen 1 bis 8, **dadurch gekennzeichnet, dass** der Ligand Tributylphosphin, (+)- oder (-)-(2,2-Dimethyl-4,5-diphenylphosphinomethyl)-1,3-dioxolan, (R)- oder (S)-BINAP und/oder (R,S)-BINAPHOS und deren Stereoisomere ist.

10. Verfahren nach Anspruch 9, **dadurch gekennzeichnet, dass** der Ligand Tributylphosphin ist und das Molverhältnis von Tributylphosphin zu Rhodium 1:1 bis 25:1, vorzugsweise 4:1 bis 10:1, beträgt.

11. Verfahren nach Anspruch 9, **dadurch gekennzeichnet, dass** der Ligand (R)- oder (S)-BINAP ist und das Molverhältnis von BINAP zu Rhodium 1:1 bis 6:1, vorzugsweise 1:1 bis 2:1, beträgt.

12. Verfahren nach Anspruch 9, **dadurch gekennzeichnet, dass** der Ligand (R,S)-BINAPHOS oder dessen Stereoisomere ist und das Molverhältnis von BINAPHOS zu Rhodium 1:1 bis 6:1, vorzugsweise 1:1 bis 2:1, beträgt.

## Claims

1. Method for producing 3,3-diarylpropylamines of general formula (I) 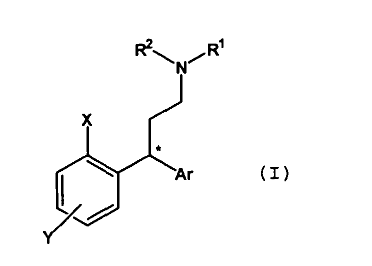 wherein
Ar represents a substituted or unsubstituted aryl radical,
X represents OH or OR³,
Y represents Cl, Br, I, CN, CHO, CH₂OR, COOH, COOR, wherein R represents C₁-C₁₀-alkyl or a substituted or unsubstituted aryl radical, or C₁-C₁₀-alkyl,
R¹, R² represents C₁-C₁₀-alkyl or C₃-C₈-cycloalkyl, wherein R¹ and R² can be linked to form a cyclic structure,
and wherein R³ represents a radical that is derived from one of the following compounds:
(i) the amino acids D-proline, L-proline, D-alanine, L-alanine, D-asparagine, L-asparagine, D-asparaginic acid, L-asparaginic acid, D-glutamine, L-glutamine, D-glutamic acid, L-glutamic acid, D-phenylalanine, L-phenylalanine, D-histidine, L-histidine, D-leucine, L-leucine, D-serine, L-serine, L-threonine, D-threonine, D-tryptophane, L-tryptophane, D-tyrosine, L-tyrosine, D-valine, L-valine, D-cysteine, L-cysteine, D-methionine, L-methionine, D-isoleucine, L-isoleucine, or the alcohols that are produced from these amino acids by reduction of the carboxylic acid function to a hydroxymethylene moiety,
(ii) the amino acids N-diphenylphosphanyl-D-alanine, N-diphenylphosphanyl-L-alanine, N-diphenylphosphanyl-D-proline, N-diphenylphosphanyl-L-proline, N-diphenylphosphanyl-D-phenylalanine, N-diphenylphosphanyl-L-phenylalanine, and the alcohols that are produced from these amino acids by reduction of the carboxylic acid function to a hydroxymethylene moiety,
(iii) α-hydroxycarboxylic acid derivatives of the general formula 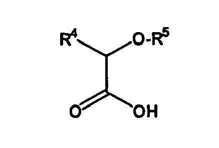 each in the form of both optical antipodes, wherein R⁴ represents a linear or branched C₁-C₁₀-alkyl group or cycloalkyl group or a substituted or unsubstituted aryl radical and R⁵ represents C₁-C₁₀-alkyl, cycloalkyl, acyl, alkoxycarbonyl, benzoyl, diphenylphosphanyl, dicyclohexylphosphanyl or diarylphosphanyl,
(iv) the compounds of the general formulae 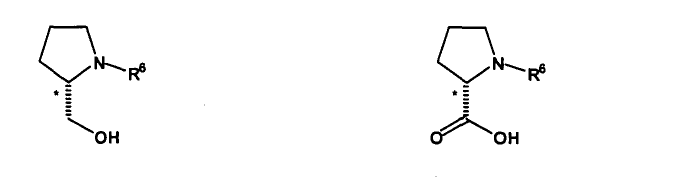 wherein R⁶ represents a substituent selected from the group consisting of PPh₂, P(C₆H₁₁)₂, P(aryl)₂, alkyl, acyl, alkoxycarbonyl, benzoyl, arylcarbonyl, diarylphosphanyl and dicyclohexylphosphanyl, and their stereoisomers,
(v) the compounds of the general formulae 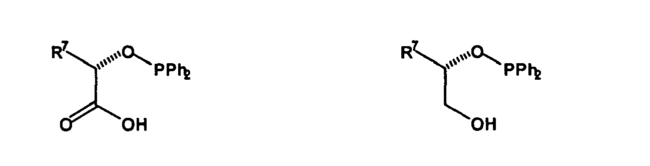 wherein R⁷ represents a linear or branched C₁-C₁₀-alkyl group or a substituted or unsubstituted aryl radical, and stereoisomers thereof,
(vi) the acids
(R)-acetoxyphenylacetic acid,
(R)- and (S)-1,4-benzodioxane-2-carboxylic acid,
(R)-(-)- and (S)-(+)-hexahydro-acetoxymandelic acid,
(2R,3S)-2,3-O-isopropylidene-2,3-dihydroxybutyric acid and stereoisomers thereof,
(+)- and (-)-menthyloxyacetic acid,
(R)- and (S)-3-phenyl-2-acyloxypropionic acid,
(R)- and (S)-acetoxymandelic acid,
(R)- and (S)-α-methoxy-α-trifluormethylphenylacetic acid,
(S)-(+)-alpha-methoxyphenylacetic acid,
(R)- and (S)-5-oxo-tetrahydrofurane-2-carboxylic acid,
and the alcohols that are produced from these acids by reduction of the carboxylic acid function to a hydroxymethylene moiety,
(vii) the compounds of the general formula 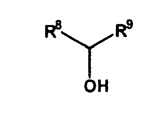 wherein R⁸ represents a substituted or unsubstituted aryl radical and R⁹ represents hydrogen or a linear or branched C₁-C₁₀-alkyl radical,
(viii) α-naphthol, β-naphthol or (R)- or (S)-1-(9-anthryl)-2,2,2-trifluoroethanol,
(ix) 2-methylamino-1-phenyl-propan-1-ol (ephedrine) in all stereomeric forms,
or R³ represents one of the following radicals:
(x) phosphite radicals of general formula -P(OR¹⁰) (OR¹¹), wherein R¹⁰ and R¹¹ can be the same or different and represent an optionally polycyclic or bridged aryl radical,
(xi) C₁-C₁₀-alkyl, branched or linear,
(xii) acyl,
(xiii) benzyl or substituted benzyl radicals,
**characterised in that** compounds of general formula (II) 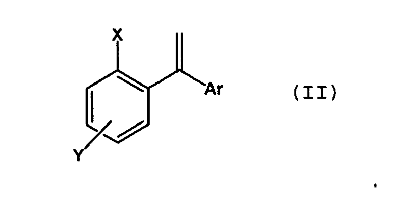 wherein X, Y and Ar are as defined above, are reacted with carbon monoxide (CO) and hydrogen (H₂) in the presence of a transition metal catalyst, and the resultant oxo compounds react in the presence of a transition metal catalyst in a one-pot reaction directly with an amine of general formula HNR¹R², R¹ and R² being defined as above, the catalyst used comprising one or more transition metals selected from the group consisting of ruthenium, rhodium, platinum, cobalt, iridium, palladium and nickel and being formed in situ from a catalyst precursor and a ligand selected from the group consisting of
(2-(diphenylphosphino)-1,1'-binaphthalen-2'-yl)-1,1'-binaphthalene-2,2'-diylphosphite (BINAPHOS),
(2,2-dimethyl-4,5-diphenylphosphinomethyl)-1,3-dioxolane, (DIOP),
(2,2-dimethyl-4,5-bis(5H-dibenzophosphol-5-ylmethyl)-1,3-dioxolane,
1,4-bis(diphenylphosphino)butane,
2,3-bis(diphenylphosphino)butane,
1,2-bis(diphenylphosphinomethyl)cyclobutane,
1,2-bis(5H-dibenzophosphol-5-ylmethyl)cyclobutane,
1,2-bis(diphenylphosphinomethyl)cyclohexane,
1,2-bis(5H-dibenzophosphol-5-ylmethyl)cyclohexane,
(2S,4S)-N-tert.-butoxycarbonyl-4-diphenylphosphino-2-diphenylphosphinomethyl-pyrrolidine (BPPM),
1,2-bis(diphenylphosphinoxy)cyclohexane, benzyl-methyl-phenylphosphine,
cyclohexyl-o-anisyl-methylphosphine, neomenthyldiphenylphosphine,
phenyl-o-anisyl-methylphosphine,
ortho-diphenylphosphanylbenzoyl,
tributylphosphine,
2,2'-bis(diphenylphosphino)-1,1'-binaphthyl (BINAP) and mixtures or stereoisomers thereof.

2. Method according to claim 1, **characterised in that** compounds of general formula (IV) 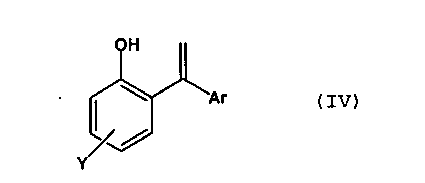 wherein Y and Ar are defined as in claim 1, are reacted with carbon monoxide (CO) and hydrogen (H₂) to a chromene/lactol system 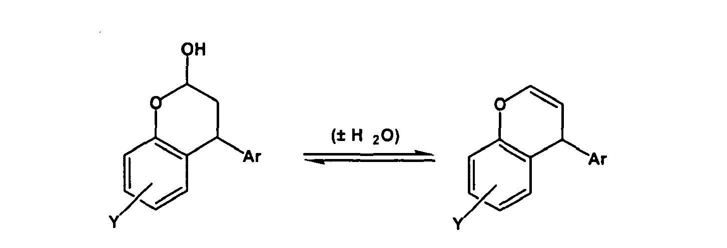 Y and Ar being defined as in claim 1, and the reaction product is allowed to react with an amine of general formula HNR¹R², R¹ and R² being defined as in claim 1.

3. Method according to claim 1 or 2, **characterised in that** it is conducted at a temperature of between 50 and 200°C, preferably between 100 and 140°C.

4. Method according to claims 1 to 3, **characterised in that** it is conducted at a pressure of between 40 and 200 bar, preferably between 80 and 120 bar.

5. Method according to claim 4, **characterised in that** the pressure ratio CO/H₂ is between 10/90 and 90/10, preferably between 70/30 and 90/10.

6. Method according to claims 1 to 5, **characterised in that** the reaction is conducted over a'period of between 2 hours and 4 days, preferably between 1 and 3 days.

7. Method according to claims 1 to 6, **characterised in that** the catalyst comprises rhodium.

8. Method according to claims 1 to 7, **characterised in that** the catalyst precursor is [Rh(cod)Cl]₂ and/or Rh(acac)(CO)₂.

9. Method according to claims 1 to 8, **characterised in that** the ligand is tributylphosphine, (+)- or (-)-(2,2-dimethyl-4,5-diphenylphosphinomethyl)-1,3-dioxolane, (R)- or (S)-BINAP and/or (R,S)-BINAPHOS and their stereoisomers.

10. Method according to claim 9, **characterised in that** the ligand is tributylphosphine and the molar ratio of tributylphosphine to rhodium is between 1:1 and 25:1, preferably between 4:1 and 10:1.

11. Method according to claim 9, **characterised in that** the ligand is (R)- or (S)-BINAP and the molar ratio of BINAP to rhodium is between 1:1 and 6:1, preferably between 1:1 and 2:1.

12. Method according to claim 9, **characterised in that** the ligand is (R,S)-BINAPHOS or its stereoisomers and the molar ratio of BINAPHOS to rhodium is between 1:1 and 6:1, preferably between 1:1 and 2:1.

## Revendications

1. Procédé pour la fabrication de 3,3-diarylpropylamines selon la formule générale (I) 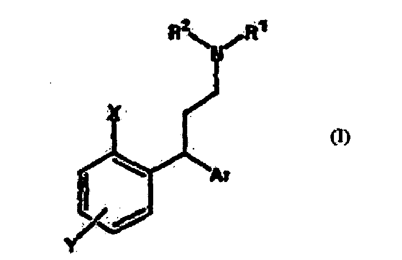 dans laquelle
Ar est un radical aryle substitué ou non substitué,
X représente OH ou OR³,
Y représente Cl, Br, I, CN, CHO, CH₂OR, COOH, COOR, où R représente un alkyle en C₁-C₁₀ ou un radical aryle substitué ou non substitué, ou un alkyle en C₁-C₁₀,
R¹, R² représente un alkyle en C₁-C₁₀ ou un cycloalkyle en C₃-C₈, R¹ et R² pouvant être liés en formant une structure cyclique,
et dans laquelle R³ représente un radical issu de l'un des composés suivants :
(i) acides aminés D-proline, L-proline, D-alanine, L-alanine, D-asparagine, L-asparagine, acide D-asparaginique, acide L-asparaginique, D-glutamine, L-glutamine, acide D-glutamique, acide L-glutamique, D-phénylalanine, L-phénylalanine, D-histidine, L-histidine, D-leucine, L-leucine, D-sérine, L-sérine, L-thréonine, D-thréonine, D-tryptophane, L-tryptophane, D-tyrosine, L-tyrosine, D-valine, L-valine, D-cystéine, L-cystéine, D-méthionine, L-méthionine, D-isoleucine, L-isoleucine, ou les alcools issus de ces acides aminés par réduction de la fonction acide carboxylique en unité hydroxyméthylène,
(ii) acides aminés N-diphénylphosphanyl-D-alanine, N-diphénylphosphanyl-L-alanine, N-diphénylphosphanyl-D-proline, N-diphénylphosphanyl-L-proline, N-diphénylphosphanyl-D-phénylalanine, N-diphénylphosphanyl-L-phénylalanine, ainsi que les alcools issus de ces acides aminés par réduction de la fonction acide carboxylique en unité hydroxyméthylène,
(iii) dérivés de l'acide α-hydroxycarboxylique selon la formule générale 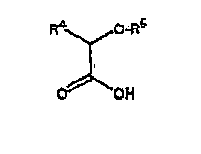 respectivement sous la forme des deux antipodes optiques, où R⁴ désigne un groupement alkyle en C₁-C₁₀ linéaire ou ramifié ou un groupement cycloalkyle ou un radical aryle substitué ou non substitué et R⁵ désigne un alkyle en C₁-C₁₀, cycloalkyle, acyle, alkoxycarbonyle, benzoyle, diphénylphosphanyle, dicyclohexylphosphanyle ou diarylphosphanyle,
(iv) composés selon les formules générales 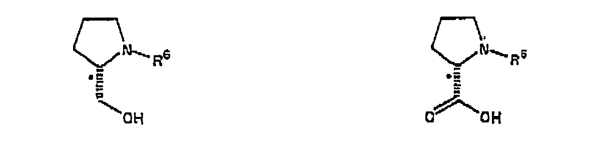 où R⁶ est choisi pour servir de substituant parmi PPh₂, P(C₆H₁₁)₂, P(aryle)₂, alkyle, acyle, alkoxycarbonyle, benzoyle, arylcarbonyle, diarylphosphanyle et dicyclohexylphosphanyle, et leurs stéréo-isomères,
(v) composés selon les formules générales 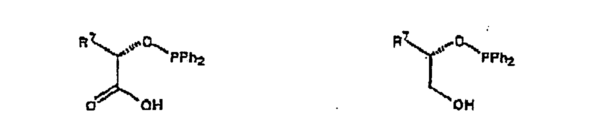 où R⁷ désigne un groupement alkyle en C₁-C₁₀ linéaire ou ramifié ou un radical aryle substitué ou non substitué et les stéréo-isomères de ceux-ci,
(vi) acides
(R)-acétoxyphénylacétique,
(R)- et (S)-1,4-benzodioxane-2-carboxylique
(R)-(-)- et (S)-(+)-hexahydro-acétoxymandélique,
(2R,3S)-2,3-O-isopropylidène-2,3-dihydroxybutyrique et ses stéréo-isomères,
(+)- et (-)-menthyloxyacétique,
(R)- et (S)-3-phényl-2-acyloxypropionique,
(R)- et (S)-acétoxymandélique,
(R)- et (S)-α-méthoxy-α-trifluorométhylphénylacétique,
(S)-(+)-α-méthoxyphénylacétique,
(R)- et (S)-5-oxo-tétrahydrofurane-2-carbonique,
ainsi que les alcools issus de ces acides par réduction de la fonction acide carboxylique en unité hydroxyméthylène,
(vii) composés selon la formule générale 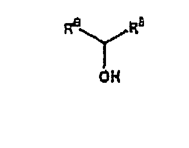 où R⁸ désigne un radical aryle substitué ou non substitué et R⁹ désigne l'hydrogène ou un radical alkyle en C₁-C₁₀ linéaire ou ramifié,
(viii) α-naphtol, β-naphtol ou (R)- ou (S)-1-(9-anthryl)-2,2,2-trifluoroéthanol,
(ix) 2-méthylamino-1-phényl-propan-1-ol (éphédrine) sous toutes ses formes stéréomères,
ou R³ désigne l'un des radicaux suivants :
(x) radicaux phosphite selon la formule générale -P(OR¹⁰)(OR¹¹), où R¹⁰ et R¹¹ peuvent être identiques ou différents et désignent un radical aryle éventuellement polycyclique ou ponté,
(xi) alkyle en C₁-C₁₀ ramifié ou linéaire,
(xii) acyle,
(xiii) benzyle ou radicaux benzyle substitués,
***caractérisé en ce que*** les composés selon la formule générale (II), 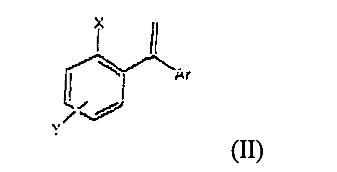 dans laquelle X, Y et Ar sont tels que définis plus haut, sont convertis ensemble avec du monoxyde de carbone (CO) et de l'hydrogène (H₂) en présence d'un catalyseur aux métaux de transition et les composés oxo en résultant sont mis en réaction directement en présence d'un catalyseur aux métaux de transition, dans une réaction monotope, avec une amine selon la formule générale HNR¹R², dans laquelle R¹ et R² sont tels que définis plus haut, le catalyseur utilisé étant composé d'un ou plusieurs métaux de transition choisis dans le groupe comprenant le ruthénium, le rhodium, le platine, le cobalt, l'iridium, le palladium et le nickel, et étant formé *in situ* à partir d'un précurseur de catalyseur et d'un ligand choisi dans le groupe comprenant :
(2-(diphénylphosphino)-1,1'-binaphtalène-2'-yl)-1,1'-binaphtalène-2,2'-diyl-phosphite (BINAPHOS),
(2,2-diméthyl-4,5-diphénylphosphinométhyl)-1,3-dioxolane (DIOP),
(2,2-diméthyl-4,5-bis(5H-dibenzophosphol-5-ylméthyl)-1,3-dioxolane,
1,4-bis(diphénylphosphino)butane,
2,3-bis(diphénylphosphino)butane,
1,2-bis(diphénylphosphinométhyl)cyclobutane,
1,2-bis(5H-dibenzophosphol-5-ylméthyl)cyclobutane,
1,2-bis(diphénylphosphinométhyl)cyclohexane,
1,2-bis(5H-dibenzophosphol-5-ylméthyl)cyclohexane,
(2S,4S)-N-tert.-butoxycarbonyl-4-diphénylphosphino-2-diphénylphosphinométhylpyrrolidine (BPPM),
1,2-bis (diphénylphosphinoxy)cyclohexane,
benzyl-méthyl-phénylphosphine,
cyclohexyl-o-anisyl-méthylphosphine,
néo-menthyldiphénylphosphine,
phényl-o-anisyl-méthylphosphine,
ortho-diphénylphosphanylbenzoyle,
tributylphosphine,
2,2'-bis(diphénylphosphino)-1,1'-binaphtyle (BINAP)
et des mélanges ou bien des stéréo-isomères de ceux-ci.

2. Procédé selon la revendication 1, ***caractérisé en ce que*** des composés selon la formule générale (IV) 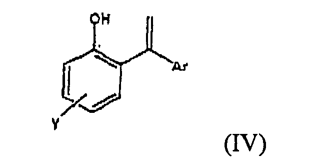 dans laquelle Y et Ar sont tels que définis dans la revendication 1, sont convertis avec du monoxyde de carbone (CO) et de l'hydrogène (H₂) en un système chromène/lactol 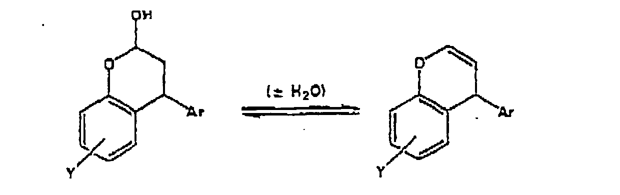 dans lequel Y et Ar sont tels que définis dans la revendication 1 et le produit de conversion est converti avec une amine selon la formule générale HNR¹R² où R¹ et R² sont tels que définis dans la revendication 1.

3. Procédé selon la revendication 1 ou 2, ***caractérisé en ce qu*'**il est mis en oeuvre à une température comprise entre 50 et 200°C, de préférence entre 100 et 140°C.

4. Procédé selon les revendications 1 à 3, ***caractérisé en ce qu*'**il est mis en oeuvre à une pression comprise entre 40 et 200 bars, de préférence entre 80 et 120 bars.

5. Procédé selon la revendication 4, ***caractérisé en ce que*** le rapport de pression CO/H₂ est compris entre 10/90 et 90/10, de préférence entre 70/30 et 90/10.

6. Procédé selon les revendications 1 à 5, ***caractérisé en ce que*** la réaction est conduite sur une durée de 2 heures à 4 jours, de préférence de 1 à 3 jours.

7. Procédé selon les revendications 1 à 6, ***caractérisé en ce que*** le catalyseur contient du rhodium.

8. Procédé selon les revendications 1 à 7, ***caractérisé en ce que*** le précurseur de catalyseur est du [Rh(cod)Cl]₂ et/ou du Rh(acac)(CO)₂.

9. Procédé selon les revendications 1 à 8, ***caractérisé en ce que*** le ligand est de la tributylphosphine, du (+)- ou (-)-(2,2-diméthyl-4,5-diphénylphosphinométhyl)-1,3-dioxolane, du (R)- ou (S)-BINAP et/ou du (R,S)-BINAPHOS et leurs stéréo-isomères.

10. Procédé selon la revendication 9 ***caractérisé en ce que*** le ligand est de la tributylphosphine et le rapport molaire entre la tributylphosphine et le rhodium est compris entre 1/1 et 25/1, de préférence entre 4/1 et 10/1.

11. Procédé selon la revendication 9, ***caractérisé en ce que*** le ligand est du (R)- ou (S)-BINAP et le rapport molaire entre le BINAP et le rhodium est compris entre 1/1 et 6/1, de préférence entre 1/1 et 2/1.

12. Procédé selon la revendication 9, ***caractérisé en ce que*** le ligand est du (R,S)-BINAPHOS ou ses stéréo-isomères et le rapport molaire entre le BINAPHOS et le rhodium est compris entre 1/1 et 6/1, de préférence entre 1/1 et 2/1.
